(19) Europäisches Patentamt
European Patent Office
Office européen des brevets

(11) **EP 4 321 859 B1**

(12) **EUROPÄISCHE PATENTSCHRIFT**

(45) Veröffentlichungstag und Bekanntmachung des Hinweises auf die Patenterteilung:
**12.03.2025 Patentblatt 2025/11**

(21) Anmeldenummer: **23186267.3**

(22) Anmeldetag: **19.07.2023**

(51) Internationale Patentklassifikation (IPC):
***G01N 27/16*** *(2006.01)* ***G01N 33/22*** *(2006.01)*
***G01N 25/32*** *(2006.01)*

(52) Gemeinsame Patentklassifikation (CPC):
**G01N 27/16; G01N 25/32; G01N 33/225**

(54) **GASMESSVORRICHTUNG UND GASMESSVERFAHREN ZUR MESSUNG EINER ZIELGAS-KONZENTRATION UND EINER UMGEBUNGSFEUCHTE**

GAS MEASURING DEVICE AND GAS MEASURING METHOD FOR MEASURING TARGET GAS CONCENTRATION AND AMBIENT HUMIDITY

DISPOSITIF DE MESURE DES GAZ ET MÉTHODE DE MESURE DES GAZ POUR MESURER LA CONCENTRATION DES GAZ CIBLES ET L'HUMIDITÉ AMBIANTE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **10.08.2022 DE 102022120102**

(43) Veröffentlichungstag der Anmeldung:
**14.02.2024 Patentblatt 2024/07**

(73) Patentinhaber: **Dräger Safety AG & Co. KGaA**
**23560 Lübeck (DE)**

(72) Erfinder:
• **OSSWALD, Jürgen**
**23558 Lübeck (DE)**
• **PÖTHIG, Tom**
**23558 Lübeck (DE)**
• **RUGE, Jan Philipp**
**23558 Lübeck (DE)**

(74) Vertreter: **Meyer-Gramann, Klaus Dieter**
**Drägerwerk Safety AG & Co. KGaA**
**Revalstraße 1**
**23560 Lübeck (DE)**

(56) Entgegenhaltungen:
DE-A1- 4 130 099     DE-B4- 102006 059 566
US-A1- 2008 282 771

# EP 4 321 859 B1

**Beschreibung**

Die Erfindung betrifft eine Gasmessvorrichtung (Anspruch 1) und ein Gasmessverfahren (Anspruch 6), welche die Konzentration eines brennbaren Zielgases zu messen vermögen. Bekannt geworden sind Gasmessvorrichtungen mit einem Detektor, der im Betrieb erhitzt wird. Der Detektor oxidiert ein brennbares Zielgas, wodurch Wärmeenergie freigesetzt wird - natürlich nur, wenn ausreichend viel brennbares Zielgas in der Umgebung und damit im Inneren des Detektors vorhanden ist. Die freigesetzte Wärmeenergie korreliert mit der Zielgas-Konzentration und verändert eine Eigenschaft des Detektors, beispielsweise den elektrischen Widerstand. Ein Maß für diese Eigenschaft wird gemessen. Eine derartige Gasmessvorrichtung wird auch als Wärmetönungs-Sensor bezeichnet. Auch die erfindungsgemäße Gasmessvorrichtung nutzt dieses Prinzip.

Die Messergebnisse einer solchen Gasmessvorrichtung werden aber der Regel nicht nur von brennbarem Zielgas beeinflusst, sondern auch von Umgebungsbedingungen. Daher umfasst eine Gasmessvorrichtung häufig zusätzlich zum Detektor einen Kompensator, wobei der Kompensator kein oder weniger Zielgas oxidiert, aber den Umgebungsbedingungen idealerweise genauso wie der Detektor ausgesetzt ist und genauso reagiert. Auch die erfindungsgemäße Gasmessvorrichtung umfasst einen solchen Kompensator.

In DE 38 08 305 C2 wird ein Gasmessgerät beschrieben, welches die Konzentration eines brennbaren Zielgases, beispielsweise Methan, zu messen vermag. Das Gasmessgerät umfasst eine Messbrücke 1, die einen Sensor für die Wärmeleitfähigkeit 2 umfasst, eine Messbrücke 3 mit einem Temperatursensor 4 und eine Messeinrichtung 5 für die Feuchte. Ein Feuchtesensor 6 der Messeinrichtung 5 umfasst in einer Realisierungsform einen Kondensator, dessen Kapazität von der aufgenommenen Feuchte abhängt. Die Zielgas-Konzentration wird aus einem Signal der Messbrücke 1 hergeleitet. Die gemessene Temperatur und die gemessene Feuchte werden verwendet, um den Einfluss der Temperatur und den der Feuchte auf das Signal der Messbrücke 1 zu kompensieren.

Das Gasmessgerät von DE 41 30 099 A1 saugt eine Gasprobe in eine Verbrennungskammer. Eine Messbrücke 1 umfasst einen katalytisch aktiven Messsensor 2 als Messelement und einen katalytisch inaktiven Sensor 3 als Kompensationselement, außerdem zwei Festwiderstände 4 und 5. Ein Temperatursensor 8 in einem Messkopf 7 im Inneren des Gasmessgeräts misst die Temperatur, so dass der Einfluss wechselnder Umgebungstemperaturen sowie unterschiedlicher Temperaturen in den beiden Brennkammerräumen 7a sich kompensieren lässt.

DE 10 2007 021 957 A1 zeigt einen Gassensor mit einem Detektierelement 7 in Form einer Detektierperle und einem Kompensatorelement 8 in Form einer Kompensatorperle. Die Detektierperle 7 ist mit einem katalytischen Material beschichtet, die Kompensatorperle 8 nicht. Die beiden Elemente 7, 8 sind in einer elektrischen Brückenschaltung in Form einer Wheatsone'schen Messbrücke angeordnet, die zusätzlich Gleichgewichtswiderstände R1, 20, 22 sowie einen Abgleichwiderstand 24 umfasst. Der Abgleichwiderstand 24 hält die Brückenschaltung im Gleichgewicht, solange kein brennbares Zielgas vorhanden ist. Der Vorgang, dass die Detektierperle 7 brennbares Zielgas oxidiert, verändert den Widerstand von Leitungen in der Brückenschaltung, und ein Voltmeter 26 misst ein Signal. Die Spannung an der Kompensatorperle 8 kann sich unabhängig von der Spannung an der Detektierperle 7 ändern. Ein Voltmeter 30 misst die Spannung an der Kompensatorperle 8, und diese Spannung ist ein Maß für die Umgebungstemperatur.

US 2008/282771 A1 offenbart eine Vorrichtung und ein Verfahren zum Detektieren eines brennbaren Gases. Eine Feuchtigkeitsabweichung wird auf der Grundlage einer Korrelation zwischen einer voreingestellten Gaskonzentration des brennbaren Gases und der berechneten Feuchtigkeitsabweichung berechnet, eine berechnete Feuchtigkeit der zu detektierenden Atmosphäre wird unter Verwendung der berechneten Feuchtigkeitsabweichung korrigiert, um eine korrigierte Feuchtigkeit zu erhalten. Wenn eine Konzentration des brennbaren Gases auf der Grundlage einer Klemmspannung, einer Temperatur der zu erfassenden Atmosphäre und einer Feuchtigkeit der zu erfassenden Atmosphäre berechnet wird, wird die berechnete korrigierte Feuchtigkeit für die Feuchtigkeit der zu erfassenden Atmosphäre verwendet. Diese wird auf der Grundlage der Klemmspannungen an den ersten und zweiten Heizwiderständen und der ermittelten Temperatur berechnet.

Der Erfindung liegt die Aufgabe zugrunde, eine Gasmessvorrichtung bereitzustellen, welche die Konzentration eines brennbaren Zielgases zu messen vermag und bei annähernd gleicher Zuverlässigkeit einfacher aufgebaut ist als bekannte Gasmessvorrichtungen. Außerdem liegt der Erfindung die Aufgabe zugrunde, ein Gasmessverfahren unter Verwendung einer solchen Gasmessvorrichtung bereitzustellen.

Die Aufgabe wird durch eine Gasmessvorrichtung mit den Merkmalen des Anspruchs 1 und durch ein Gasmessverfahren mit den Merkmalen des Anspruchs 6 gelöst. Vorteilhafte Ausgestaltungen der erfindungsgemäßen Gasmessvorrichtung sind, soweit sinnvoll, auch vorteilhafte Ausgestaltungen des erfindungsgemäßen Gasmessverfahrens und umgekehrt.

Die erfindungsgemäße Gasmessvorrichtung und das erfindungsgemäße Gasmessverfahren vermögen die Konzentration eines brennbaren Zielgases zu messen. Dieses brennbare Zielgas tritt in der Umgebung der Gasmessvorrichtung auf, insbesondere in einem zu überwachenden räumlichen Bereich. Diese Umgebung befindet sich beispielsweise in einer chemischen Fertigungsanlage oder ist ein umschlossener Raum eines Gebäudes oder das Innere eines Fahrzeugs.

Möglich ist, dass sich in der Umgebung gleichzeitig mehrere brennbare Zielgase befinden. In einer Ausgestaltung wird unter "dem brennbaren Zielgas" eines dieser Zielgase verstanden. In einer anderen Ausgestaltung wird unter der gemessenen Zielgas-Konzentration die Summe der Konzentrationen aller vorhandenen brennbaren Zielgase verstanden.

Die Gasmessvorrichtung umfasst einen Detektor mit einem heizenden Detektor-Segment und einen Kompensator mit einem heizenden Kompensator-Segment. Wenigstens zeitweise ist zwischen dem Detektor und dem Kompensator einerseits und der Umgebung der Gasmessvorrichtung andererseits eine Fluidverbindung hergestellt. Eine Gasprobe aus der Umgebung kann durch diese Fluidverbindung hindurch zeitgleich sowohl den Detektor als auch den Kompensator erreichen. Bevorzugt ist diese Fluidverbindung unterbrochen, wenn die Gasmessvorrichtung nicht verwendet wird.

Die Gasmessvorrichtung vermag sowohl an den Detektor als auch an den Kompensator jeweils eine elektrische Spannung anzulegen. Die beiden anliegenden elektrischen Spannungen können stets übereinstimmen oder wenigstens zeitweise voneinander abweichen. In einer bevorzugten Realisierungsform werden die elektrischen Spannungen gepulst angelegt.

Wenn eine elektrische Spannung am Detektor angelegt ist, fließt ein elektrischer Strom durch das heizende Detektor-Segment, wodurch das heizende Detektor-Segment erhitzt wird. Wenn eine elektrische Spannung am Kompensator angelegt ist, fließt ein elektrischer Strom durch das heizende Kompensator-Segment, wodurch das heizende Kompensator-Segment erhitzt wird.

Der Detektor vermag Folgendes zu bewirken: Eine Erhitzung des heizenden Detektor-Segments bewirkt, dass brennbares Zielgas oxidiert wird, wobei dieses brennbare Zielgas sich im Inneren der Gasmessvorrichtung befindet. Dieser Effekt kann natürlich nur dann eintreten, wenn brennbares Zielgas mit einer ausreichend hohen Konzentration in der Umgebung und daher auch im Inneren der Gasmessvorrichtung vorhanden ist.

In einer ersten Alternative vermag der Kompensator konstruktionsbedingt trotz Erhitzung des Kompensator-Segments in geringerem Umfang als der Detektor oder sogar überhaupt nicht das brennbare Zielgas zu oxidieren. In einer zweiten Alternative ist die Gasmessvorrichtung folgt ausgestaltet: Aus einer Umgebung der Gasmessvorrichtung fließt pro Zeiteinheit eine geringere Menge des brennbaren Zielgases zu dem Kompensator als zu dem Detektor. Diese beiden Alternativen können gleichzeitig (in Kombination) realisiert sein.

Die Gasmessvorrichtung umfasst weiterhin eine Sensor-Anordnung. Die Sensor-Anordnung vermag eine erste Detektionsgröße und eine zweite Detektionsgröße zu messen, bevorzugt wiederholt zu messen. Die zweite Detektionsgröße ist von der ersten Detektionsgröße verschieden, und die beiden Detektionsgrößen können unabhängig voneinander über der Zeit variieren oder voneinander abhängen. Bevorzugt hängt die eine Detektionsgröße nicht von der anderen Detektionsgröße ab.

In einer ersten Alternative hängt die erste Detektionsgröße von der Detektor-Temperatur ab, aber bevorzugt nicht von der Kompensator-Temperatur. Die zweite Detektionsgröße hängt von der Kompensator-Temperatur ab, aber bevorzugt nicht von der Detektor-Temperatur. Unter der "Detektor-Temperatur" wird die Temperatur des heizenden Detektor-Segments verstanden, unter der "Kompensator-Temperatur" die Temperatur des heizenden Kompensator-Segments. In einer zweiten Alternative hängt die erste Detektionsgröße sowohl von der Detektor-Temperatur als auch von der Kompensator-Temperatur ab, insbesondere von der Differenz zwischen der Detektor-Temperatur und der Kompensator-Temperatur. Die zweite Detektionsgröße hängt entweder von der Detektor-Temperatur, aber nicht von der Kompensator-Temperatur, oder von der Kompensator-Temperatur, aber nicht von der Detektor-Temperatur ab.

Weiterhin umfasst die Gasmessvorrichtung einen Temperatur-Sensor. Der Temperatur-Sensor vermag ein Maß für die Temperatur in der Umgebung der Gasmessvorrichtung zu messen, bevorzugt wiederholt zu messen. Die Messposition, an der Temperatur-Sensor das Maß für die Umgebungstemperatur misst, kann innerhalb oder außerhalb eines Gehäuses der Gasmessvorrichtung liegen. Idealerweise beeinflussen weder die Detektor-Temperatur noch die Kompensator-Temperatur die Messung der Umgebungstemperatur, was beispielsweise durch eine geeignete thermische Isolierung zwischen dem Detektor und dem Kompensator einerseits und den Temperatur-Sensor andererseits erreicht wird. Möglich ist auch, dass die Gasmessvorrichtung ein Signal mit einer Information über die Umgebungstemperatur von einem räumlich entfernten Temperatur-Sensor empfängt. Der Temperatur-Sensor ist dann nicht notwendigerweise ein Bestandteil der Gasmessvorrichtung. Dieses Signal mit der Umgebungstemperatur kann an mehrere erfindungsgemäße Gasmessvorrichtungen übermittelt werden.

**In** einem Datenspeicher der Gasmessvorrichtung ist ein rechnerauswertbares Modell abgespeichert. Dem erfindungsgemäßen Gasmessverfahren wird dieses Modell vorgegeben. Das Modell wurde vor einem Einsatz der Gasmessvorrichtung erzeugt. "Rechnerauswertbar" bedeutet, dass ein Programm bei seiner Ausführung dieses Modell automatisch auswerten und / oder anwenden kann.

Dieses abgespeicherte Modell umfasst einen ersten funktionalen Zusammenhang und einen zweiten funktionalen Zusammenhang. Der erste funktionale Zusammenhang beschreibt eine Abhängigkeit zwischen der Zielgas-Konzentration einerseits und der ersten Detektionsgröße andererseits. Optional treten im ersten funktionalen Zusammenhang zusätzlich die Umgebungstemperatur und / oder die zweite Detektionsgröße auf, und die Zielgas-Konzentration hängt gemäß dem ersten funktionalen Zusammenhang zusätzlich von der Umgebungstemperatur und / oder der zweiten Detektionsgröße ab.

Der zweite funktionale Zusammenhang beschreibt eine Abhängigkeit zwischen einer ersten weiteren Umgebungsbedingung einerseits und der Umgebungstemperatur sowie der ersten Detektionsgröße und / oder der zweiten Detektionsgröße andererseits. Die erste weitere Umgebungsbedingung ist von der Umgebungstemperatur verschieden. Möglich ist, dass die Zielgas-Konzentration gemäß dem ersten funktionalen Zusammenhang zusätzlich von der ersten weiteren Umgebungsbedingung abhängt.

Insbesondere ist die erste weitere Umgebungsbedingung die Umgebungsfeuchte oder der Umgebungsdruck sowie die Konzentration von Kohlendioxid oder eines sonstigen für Menschen nicht schädlichen Gases in der Umgebungsluft. Möglich ist auch, dass die erste weitere Umgebungsbedingung den summierten Einfluss aller derjenigen weiteren Umgebungsbedingungen beschreibt, die nicht durch einen Sensor gemessen werden, also insbesondere den summierten Einfluss von Umgebungsfeuchte und Umgebungsdruck. Der Ansatz, alle nicht direkt gemessenen weiteren Umgebungsbedingungen durch die erste weitere Umgebungsbedingung zu beschreiben und die erste weitere Umgebungsbedingung zu ermitteln, ist insbesondere dann gerechtfertigt, wenn mit ausreichender Genauigkeit angenommen werden kann, dass sowohl die Detektor-Temperatur als auch die Kompensator-Temperatur jeweils linear von jeder nicht direkt gemessenen weiteren Umgebungsbedingung abhängt. **In** der Praxis ist diese Annahme häufig zutreffend.

Sowohl die Umgebungstemperatur als auch die oder jede weitere Umgebungsbedingung treten in der Regel unabhängig vom Vorhandensein und der Konzentration eines brennbaren Zielgases auf und sind in der Regel nicht schädlich für einen Menschen, zumindest nicht bei einer ausreichend geringen Konzentration.

Der zweite funktionale Zusammenhang ist mindestens dann gültig, beschreibt also dann ausreichend genau die physikalischen Realität, wenn die Konzentration des Zielgases in der Umgebung und daher im Inneren der Gasmessvorrichtung unter einer vorgegebenen Konzentrations-Schranke liegt.

Bei einer größeren Zielgas-Konzentration ist der zweite funktionale Zusammenhang nicht mehr notwendigerweise gültig, wohl aber der erste funktionale Zusammenhang.

Eine signalverarbeitende Auswerteeinheit der Gasmessvorrichtung hat wenigstens zeitweise Lesezugriff auf den Datenspeicher mit dem Modell. Die Auswerteeinheit vermag die Konzentration des Zielgases wenigstens näherungsweise zu ermitteln - genauer gesagt: einen Wert für die Zielgas-Konzentration herzuleiten. Um die Zielgas-Konzentration zu ermitteln, verwendet die Auswerteeinheit die gemessene erste Detektionsgröße sowie optional die gemessene Umgebungstemperatur und / oder die gemessene zweite Detektionsgröße. Die Auswerteeinheit wendet den abgespeicherten ersten funktionalen Zusammenhang an, um die Zielgas-Konzentration zu ermitteln. In der Regel vermag die Auswerteeinheit die Zielgas-Konzentration nur näherungsweise zu ermitteln.

Die Auswerteeinheit vermag automatisch zu entscheiden, ob der zweite funktionale Zusammenhang bei der ermittelten Zielgas-Konzentration gültig ist oder nicht. Bevorzugt vergleicht die Auswerteeinheit für diese Entscheidung die ermittelte Konzentration des Zielgases in der Umgebung der Gasmessvorrichtung mit der vorgegebenen Konzentrations-Schranke.

Weiterhin ist die Auswerteeinheit dazu ausgestaltet, den folgenden Schritt durchzuführen: Falls die Auswerteeinheit entschieden hat, dass der zweite funktionale Zusammenhang aktuell gültig ist, ermittelt die Auswerteeinheit die erste weitere Umgebungsbedingung - genauer: leitet einen Wert für die erste weitere Umgebungsbedingung her. Um die erste weitere Umgebungsbedingung zu ermitteln, verwendet die Auswerteeinheit die gemessene Umgebungstemperatur sowie die erste gemessene Detektionsgröße und / oder die zweite gemessene Detektionsgröße, optional beide Detektionsgrößen. Genauer gesagt: Um die erste weitere Umgebungsbedingung zu ermitteln, verwendet die Auswerteeinheit einen Wert für die erste Detektionsgröße und / oder einen Wert für die zweite Detektionsgröße, wobei der oder jeder verwendete Wert gemessen worden ist, als die Zielgas-Konzentration unterhalb der Konzentrations-Schranke lag. Die Auswerteeinheit wendet den zweiten funktionalen Zusammenhang an, um die erste weitere Umgebungsbedingungen zu ermitteln.

Erfindungsgemäß oxidiert der Detektor ein brennbares Zielgas im Inneren der Gasmessvorrichtung - natürlich nur dann, wenn eine ausreichende Menge von brennbarem Zielgas im Inneren vorhanden ist. Beim Oxidieren wird Wärmeenergie freigesetzt, und die freigesetzte Wärmeenergie wirkt auf das heizende Detektor-Segment ein. Dadurch erhöht die freigesetzte Wärmeenergie die Temperatur des heizenden Detektor-Segments verglichen mit einem Zustand ohne brennbarem Zielgas. Diese Temperatur-Erhöhung ist ein Maß für die Konzentration von brennbarem Zielgas im Inneren der Gasmessvorrichtung und damit in einer Umgebung der Gasmessvorrichtung. Mindestens eine der beiden Detektionsgrößen hängt von der Detektor-Temperatur ab, also von der Temperatur des heizenden Detektor-Segments. Bekanntlich verändert bei vielen elektrisch leitenden Materialien die Temperatur den elektrischen Widerstand, und mindestens eine Detektionsgröße hängt von der Temperatur und daher vom elektrischen Widerstand des heizenden Detektor-Segments ab.

Die Detektor-Temperatur wird aber nicht nur von der Wärmeenergie beeinflusst, die beim Oxidieren von brennbarem Zielgas freigesetzt wird, sondern in der Regel auch von der Umgebungstemperatur und mindestens einer weiteren Umgebungsbedingung. Die Umgebungstemperatur wird vom Temperatur-Sensor gemessen, und in einer Ausgestaltung verwendet die Auswerteeinheit die gemessene Umgebungstemperatur, um die Zielgas-Konzentration zu ermitteln. **In** vielen Fällen beeinflusst die Umgebungstemperatur die Detektor-Temperatur stärker als jede weitere Umgebungsbedingung. Daher erhöht das Merkmal, dass die Gasmessvorrichtung einen Temperatur-Sensor umfasst, die Zuverlässigkeit der Gasmessvorrichtung, verglichen mit einer Gasmessvorrichtung ohne eigenen Temperatur-Sensor.

Die erfindungsgemäße Gasmessvorrichtung vermag den Einfluss der oder mindestens einer weiteren Umgebungsbedingung auf die Ermittlung der Zielgas-Konzentration bis zu einem gewissen Grad konstruktiv und / oder rechnerisch zu kompensieren. Diese Wirkung erzielt die Gasmessvorrichtung dadurch, dass die Gasmessvorrichtung zusätzlich zum Detektor einen Kompensator mit einem heizenden Kompensator-Segment umfasst. Die erste Detektionsgröße und / oder die zweite Detektionsgröße hängen von der Kompensator-Temperatur ab. Der erste funktionale Zusammenhang ist in einer Realisierungsform so ausgestaltet, dass die oder jede weitere Umgebungsbedingung die Zielgas-Konzentration, die mittels des ersten funktionalen Zusammenhangs ermittelt wird, nur in nicht relevanter Weise, idealerweise überhaupt nicht, beeinflusst. Dies ist häufig möglich, weil die oder jede weitere Umgebungsbedingung sich idealerweise gleichartig auf den Detektor und auf den Kompensator auswirkt.

Erfindungsgemäß werden zwei verschiedene Detektionsgrößen sowie die Umgebungstemperatur von der Gasmessvorrichtung gemessen. Die Auswerteeinheit verwendet mindestens Messwerte dieser drei Größen sowie die beiden abgespeicherten funktionalen Zusammenhänge, um einerseits die Zielgas-Konzentration und andererseits die erste weitere Umgebungsbedingung zu ermitteln. Somit werden zwei verschiedene funktionale Zusammenhänge angewendet, um zwei Unbekannte, nämlich die Zielgas-Konzentration und die erste weitere Umgebungsbedingung, zu ermitteln.

Die Erfindung zeigt einen Weg auf, um die erste weitere Umgebungsbedingung zu ermitteln, ohne einen Sensor für die erste weitere Umgebungsbedingung zu benötigen. Ein Umgebungsbedingungs-Sensor ist häufig zwangsläufig relativ groß und / oder schwer und verbraucht in der Regel elektrische Energie. Außerdem muss ein solcher Sensor in der Regel mindestens vor dem Einsatz justiert und im Einsatz überwacht werden. Daher ist es von Vorteil, nicht notwendigerweise einen solchen Umgebungsbedingungs-Sensor verwenden zu müssen, der ein Bestandteil der Gasmessvorrichtung ist. Die Erfindung stellt eine Gasmessvorrichtung bereit, welche die erste weitere Umgebungsbedingung zu ermitteln vermag und einfacher aufgebaut ist als eine Gasmessvorrichtung, welche zusätzlich einen Sensor für die erste weitere Umgebungsbedingung umfasst.

Ausreichend ist es, den zweiten funktionalen Zusammenhang so aufzustellen, dass er mindestens dann gültig ist, wenn die Zielgas-Konzentration unterhalb der Konzentrations-Schranke liegt. Dieses Merkmal vereinfacht es in vielen Fällen, einem zweiten funktionalen Zusammenhang aufzustellen, der ausreichend genau mit der physikalischen Realität übereinstimmt, verglichen mit einem zweiten funktionalen Zusammenhang, der bei jeder Zielgas-Konzentration gültig sein soll. In der Regel führt dieses Merkmal zu einer nur relativ geringen Einschränkung. Denn meistens tritt in der Praxis die erwünschten Normalsituation auf, dass relativ selten brennbares Zielgas austritt und daher die Zielgas-Konzentration meistens unterhalb der Konzentrations-Schranke liegt. Daher vermag die erfindungsgemäße Gasmessvorrichtung in der Regel über relativ lange Zeitspannen jeweils aktuelle Werte für die erste weitere Umgebungsbedingung zu liefern, nämlich bei einer Zielgas-Konzentration unterhalb der Konzentrations-Schranke, was die Normalsituation ist. Falls eine Zielgas-Konzentration oberhalb der Konzentrations-Schranke auftritt, so lässt sich häufig ein zuvor ermittelter Wert für die erste weitere Umgebungsbedingung weiterverwenden, oder ein zuvor ermittelter zeitlicher Verlauf der ersten weiteren Umgebungsbedingung lässt sich in die Zukunft fortschreiben. Denn in aller Regel ändert sich die erste weitere Umgebungsbedingung nur relativ langsam.

In der Regel übertrifft der Einfluss, den das brennbare Zielgas aufgrund des Oxidierens auf die Detektor-Temperatur nimmt, den Einfluss der oder jeder weiteren Umgebungsbedingung auf die Detektor-Temperatur - vorausgesetzt die Zielgas-Konzentration ist größer als die oder gleich der Konzentrations-Schranke. Daher beschreibt der erste funktionale Zusammenhang die physikalischen Realität auch dann ausreichend genau, wenn im ersten funktionalen Zusammenhang die erste weitere Umgebungsbedingung nicht auftritt oder wenn im ersten funktionalen Zusammenhang für die erste weitere Umgebungsbedingung anstelle eines aktuellen Messwerts ein vorgegebener Standardwert oder ein hergeleiteter Wert aus der Vergangenheit verwendet wird. Insbesondere vermag die Auswerteeinheit die Entscheidung, ob die Zielgas-Konzentration oberhalb oder unterhalb der Konzentrations-Schranke liegt, ohne Kenntnis der ersten weiteren Umgebungsbedingung zu fällen. Das Ergebnis dieser Entscheidung wird einerseits erfindungsgemäß für die Prüfung verwendet, ob der zweite funktionale Zusammenhang aktuell gültig ist oder nicht. Andererseits lässt sich das Ergebnis dieser Entscheidung dafür verwenden, um bei einer Zielgas-Konzentration oberhalb der Konzentration eine Warnung zu generieren, wobei diese Warnung in einer von einem Menschen wahrnehmbaren Form ausgegeben wird, insbesondere visuell, akustisch oder haptisch (durch Vibrationen ).

Erfindungsgemäß wird für die Ermittlung der Zielgas-Konzentration die erste Detektionsgröße verwendet - genauer gesagt ein Wert der ersten Detektionsgröße wird verwendet. Außerdem wird erfindungsgemäß entschieden, ob der zweite funktionale Zusammenhang gültig ist oder nicht, wofür die ermittelte Zielgas-Konzentration verwendet wird. In einer Ausgestaltung werden die Zielgas-Ermittlung und / oder die Entscheidung abhängig von der gemessenen ersten Detektionsgröße - genauer gesagt abhängig von mindestens einem Wert der ersten Detektionsgröße - getroffen, ohne die zweite Detektionsgröße zu verwenden. Diese Ausgestaltung spart in vielen Fällen Rechenzeit ein.

In einer Ausgestaltung werden die Zielgas-Konzentration und die erste weitere Umgebungsbedingung mit der gleichen Abtast-Frequenz ermittelt. In einer bevorzugten alternativen Ausgestaltung wird die Zielgas-Konzentration hingegen mit einer höheren Abtast-Frequenz ermittelt als die erste weitere Umgebungsbedingung, bevorzugt mit einer mindestens doppelt so großen Abtast-Frequenz. Diese Ausgestaltung berücksichtigt die Tatsache, dass die Zielgas-Konzentration sich rasch ändern kann, insbesondere weil ein Leck aufgetreten ist, während die oder jede weitere Umgebungsbedingung sich in aller Regel nur relativ langsam ändert. Die Ausgestaltung mit den unterschiedlichen Abtast-Frequenzen ermöglicht es einerseits, rasch eine steigende Zielgas-Konzentration zu detektieren, und spart andererseits Rechenzeit und / oder Rechenkapazität ein verglichen mit einer Ausgestaltung, bei der auch die erste weitere Umgebungsbedingung mit einer hohen Abtast-Frequenz ermittelt wird.

In einer Realisierungsform der Erfindung sind sowohl der Detektor als auch der Kompensator Bestandteile einer Wheatstone'schen Messbrücke. Als erste Detektionsgröße misst die Sensor-Anordnung eine Brückenspannung zwischen dem Detektor und dem Kompensator. Die erste Detektionsgröße hängt also sowohl von der Detektor-Temperatur als auch von der Kompensator-Temperatur ab. Diese Realisierungsform verwendet eine bewährte Technik, um die Konzentration oder Menge eines brennbaren Zielgases zu messen.

In einer Ausgestaltung werden vorab für die erste Detektionsgröße und / oder für die zweite Detektionsgröße jeweils ein Nullwert (Referenzwert) ermittelt. Die Detektionsgröße nimmt diesen Nullwert an, wenn die Zielgas-Konzentration unter der Konzentrations-Schranke liegt und die Umgebungstemperatur und jede weitere Umgebungsbedingung jeweils einen Standardwert annehmen. Während eines Einsatzes der Gasmessvorrichtung wird als Wert der Detektionsgröße die Differenz aus dem gemessenen Wert der Detektionsgröße und dem vorab ermittelten Nullwert verwendet.

In einer Ausgestaltung beschreibt der abgespeicherte erste funktionale Zusammenhang die Abhängigkeit zwischen der Zielgas-Konzentration einerseits und der ersten Detektionsgröße, der ersten weiteren Umgebungsbedingung sowie optional der Umgebungstemperatur und / oder der zweiten Detektionsgröße andererseits. Gemäß dieser Ausgestaltung tritt die erste weitere Umgebungsbedingung also nicht nur im zweiten funktionalen Zusammenhang, sondern auch im ersten funktionalen Zusammenhang auf. Gemäß dieser Ausgestaltung verwendet die Auswerteeinheit einen Wert für die erste weitere Umgebungsbedingung, um unter Verwendung des ersten funktionalen Zusammenhangs die Zielgas-Konzentration zu ermitteln. Als Wert für die erste weitere Umgebungsbedingung verwendet die Auswerteeinheit hierbei entweder einen Wert, den sie unter Verwendung des zweiten funktionalen Zusammenhangs hergeleitet hat, oder einen vorgegebenen Standardwert oder mindestens einen Wert aus der Vergangenheit, optional eine Fortschreibung einer Zeitreihe. Natürlich lässt sich ein ermittelter Wert für die erste weitere Umgebungsbedingung nur dann verwenden, wenn zuvor der zweite funktionale Zusammenhang wenigstens zeitweise gültig war. Bevorzugt verwendet die Auswerteeinheit dann den Standardwert, wenn sie noch keinen Wert für die erste weitere Umgebungsbedingung ermittelt hat oder der zuletzt ermittelte Wert als nicht mehr gültig eingestuft wird, beispielsweise weil seit seiner Ermittlung eine zu große Zeitspanne verstrichen ist oder weil er als unplausibel eingestuft wird.

Die Ausgestaltung, dass die Zielgas-Konzentration zusätzlich unter Verwendung eines ermittelten Werts für die erste weitere Umgebungsbedingung ermittelt wird, steigert in vielen Fällen die Zuverlässigkeit der Gasmessvorrichtung.

Insbesondere wird die Gefahr reduziert, dass eine bestimmte erste weitere Umgebungsbedingung ein Messergebnis verfälscht.

In einer Ausgestaltung tritt im zweiten funktionalen Zusammenhang die zweite Detektionsgröße auf, aber nicht die erste Detektionsgröße. Gemäß dieser Ausgestaltung hängt die zweite Detektionsgröße von der Kompensator-Temperatur ab, aber nicht von der Detektor-Temperatur. Diese Ausgestaltung ist insbesondere aus folgenden Gründen vorteilhaft: Die Detektor-Temperatur hängt in der Regel stärker von der Zielgas-Konzentration als von der ersten weiteren Umgebungsbedingung ab. Die Kompensator-Temperatur hängt hingegen weniger als die Detektor-Temperatur von der Zielgas-Konzentration ab.

Erfindungsgemäß ermittelt die Auswerteeinheit einen Wert für die erste weitere Umgebungsbedingung. In einer Ausgestaltung ist für die erste weitere Umgebungsbedingung ein Wertebereich vorgegeben. Beispielsweise vermag die Gasmessvorrichtung nur dann ausreichend zuverlässig die Zielgas-Konzentration zu ermitteln, wenn die erste weitere Umgebungsbedingung in diesem Wertebereich liegt. Oder die erste weitere Umgebungsbedingung vermag physikalisch bedingt nur Werte im Wertebereich anzunehmen, sodass ein ermittelter Wert außerhalb des Wertebereichs physikalisch nicht möglich und daher ein Indiz für einen Messfehler ist.

Falls die Auswerteeinheit für die erste weitere Umgebungsbedingung einen Wert ermittelt hat, der außerhalb des vorgegebenen Wertebereichs liegt, so vermag die Gasmessvorrichtung gemäß dieser Ausgestaltung folgendes zu bewirken: Die Gasmessvorrichtung generiert eine Meldung, wobei diese Meldung bevorzugt eine Information über die gemessene erste weitere Umgebungsbedingung umfasst. Die Gasmessvorrichtung löst bevorzugt den Schritt aus, dass diese Meldung in einer von einem Menschen wahrnehmbaren Form ausgegeben wird. Diese Ausgabe wird in einer Realisierungsform von einer Ausgabeeinheit der Gasmessvorrichtung selber durchgeführt. In einer anderen Realisierungsform wird die Meldung an einen räumlich entfernten Empfänger übermittelt, und eine Ausgabeeinheit des räumlich entfernten Empfängers gibt die Meldung aus. Ein Wert außerhalb des Wertebereichs kann ein Indiz dafür sein, dass die Gasmessvorrichtung außerhalb eines zulässigen Einsatzbereichs verwendet wird. Oder die Gasmessvorrichtung weist einen Fehler auf, und ein Indiz für diesen Fehler wird automatisch erkannt.

Die Ausgestaltung mit dem Wertebereich für die erste weitere Umgebungsbedingung reduziert die Gefahr, dass die Gasmessvorrichtung ein falsches Ergebnis für die Zielgas-Konzentration liefert, weil die erste weitere Umgebungsbedingung außerhalb des Wertebereichs liegt, und das Messergebnis der Gasmessvorrichtung nicht als ein falsches Messergebnis erkannt wird. Auch bei dieser Ausgestaltung ist es nicht erforderlich, einen Sensor zu verwenden, der direkt die erste weitere Umgebungsbedingung misst.

Die Erfindung ermöglicht es, erspart aber die Notwendigkeit, dass die Gasmessvorrichtung von einem Umgebungsbedingungs-Sensor ein Signal empfängt, wobei der Umgebungsbedingungs-Sensor ein Maß für die erste weitere Umgebungsbedingung misst und das Signal eine Information über die gemessene erste weitere Umgebungsbedingung umfasst. In einer Ausgestaltung vermag die Gasmessvorrichtung ein solches Signal zu empfangen, und die Auswerteeinheit vermag durch Auswertung des empfangenen Signals einen Wert für die erste weitere Umgebungsbedingung herzuleiten. Dieser Umgebungsbedingungs-Sensor kann ein Bestandteil der Gasmessvorrichtung sein oder räumlich von der Gasmessvorrichtung beabstandet sein. Bei der zweiten Realisierungsform wird das Signal vom Umgebungsbedingungs-Sensor an die Gasmessvorrichtung übermittelt.

Bei dieser Ausgestaltung liegen also zwei Werte für die erste weitere Umgebungsbedingung vor, wobei diese beiden Werte sich bevorzugt auf denselben Abtast-Zeitpunkt beziehen. Der erste Wert ist von der Auswerteeinheit erfindungsgemäß unter Verwendung des zweiten funktionalen Zusammenhangs ermittelt worden. Der zweite Wert ist von der Auswerteeinheit durch Auswertung des empfangenen Signals ermittelt worden. In einer ersten Anwendung dieser Ausgestaltung vermag die Auswerteeinheit wahlweise den ersten Wert oder den zweiten Wert für die erste weitere Umgebungsbedingung zu verwenden. Beispielsweise verwendet sie den zweiten Wert, wenn ein solcher zweiter Wert vorliegt und optional ausreichend aktuell ist und / oder an einer Messposition gemessen worden ist, die sich ausreichend nahe an der Gasmessvorrichtung befindet. Ansonsten verwendet sie den ermittelten ersten Wert. In einer zweiten Anwendung dieser Ausgestaltung vergleicht die Auswerteeinheit den ersten Wert mit dem zweiten Wert. Dadurch vermag die Auswerteeinheit eine Plausibilitätsprüfung durchzuführen und beispielsweise bei einer großen Abweichung zwischen den beiden Werten eine Meldung zu generieren oder denjenigen der beiden Werte zu verwenden, der als zuverlässiger eingestuft wird. Diese beiden Anwendungen lassen sich miteinander kombinieren.

Erfindungsgemäß umfasst die Gasmessvorrichtung einen Temperatur-Sensor. In einer Ausgestaltung umfasst die Gasmessvorrichtung einen zweiten Umgebungsbedingungs-Sensor. Dieser ist dazu ausgestaltet, ein Maß für eine zweite weitere Umgebungsbedingung zu messen. Diese zweite weitere Umgebungsbedingung ist von der Umgebungstemperatur und von der ersten weiteren Umgebungsbedingung verschieden. Die zweite weitere Umgebungsbedingung tritt im ersten funktionalen Zusammenhang und / oder im zweiten funktionalen Zusammenhang auf. Beispielsweise ist die Umgebungsfeuchte die erste weitere Umgebungsbedingung, und der Umgebungsdruck ist die zweite weitere Umgebungsbedingung. Oft lässt sich der Umgebungsdruck deutlich leichter messen als die Umgebungsfeuchte. In manchen Fällen erhöht Ausgestaltung Mit dem zweiten Umgebungsbedingungs-Sensor die Zuverlässigkeit, mit der die Gasmessvorrichtung die Zielgas-Konzentration und / oder die erste weitere Umgebungsbedingung ermittelt.

Erfindungsgemäß wendet die Auswerteeinheit ein abgespeichertes rechnerauswertbares Modell an. In einer Ausgestaltung ist dieses Modell ein analytisches Modell, welches vorab aufgestellt worden ist und bevorzugt mindestens einen Modellparameter umfasst, besonders bevorzugt mehrere Modellparameter. Vor einem Einsatz der Gasmessvorrichtung wird dem oder jedem Modellparameter jeweils ein Wert zugewiesen. In einer Ausgestaltung hängt jeder funktionale Zusammenhang des Modells linear von den Detektionsgrößen und optional von Umgebungsbedingungen ab, wobei die Modellparameter Proportionalitäts-Faktoren sind.

Das Modell kann auch ein trainiertes neuronales Netz oder ein sonstiges Modell, das durch ein lernendes Verfahren erzeugt worden ist, sein. Möglich ist auch, dass beide funktionale Zusammenhänge durch jeweils ein neuronales Netz realisiert werden. Möglich ist auch, dass der eine funktionale Zusammenhang ein analytisches Modell ist und der andere funktionale Zusammenhang durch ein neuronales Netz realisiert wird. Um das Modell durch ein lernendes Verfahren aufzustellen, wird eine Stichprobe verwendet, wobei jedes Element der Stichprobe jeweils einen Wert für die erste Detektionsgröße, die zweite Detektionsgröße, die Umgebungstemperatur und die erste weitere Umgebungsbedingung umfasst, optional zusätzlich einen Wert für eine zweite weitere Umgebungsbedingung. Um die Stichprobe zu erzeugen, wird also ein Sensor für die erste weitere Umgebungsbedingung verwendet. Dieser Sensor wird aber nicht notwendigerweise für den Einsatz der Gasmessvorrichtung benötigt.

Erfindungsgemäß wendet die Auswerteeinheit ein Modell mit einem ersten und einem zweiten funktionalen Zusammenhang an. In einer Ausgestaltung vermag eine Kalibriervorrichtung dieses Modell zu erzeugen und zu bewirken, dass dieses Modell im Datenspeicher der Gasmessvorrichtung abgespeichert wird. Die Kalibriervorrichtung umfasst einen Umgebungsbedingungs-Sensor. Dieser Umgebungsbedingungs-Sensor vermag ein Maß für die erste weitere Umgebungsbedingung zu messen. Dieser Umgebungsbedingungs-Sensor wird nicht notwendigerweise für den Einsatz der Gasmessvorrichtung verwendet und ist daher nicht notwendigerweise ein Bestandteil der Gasmessvorrichtung, sondern gemäß dieser Ausgestaltung für die Erzeugung des Modells.

Die Kalibriervorrichtung vermag eine Stichprobe mit mehreren Stichprobenelementen zu erzeugen. Jedes Stichprobenelement umfasst jeweils mindestens vier Werte, nämlich einen Wert für die erste Detektionsgröße, einen Wert für die zweite Detektionsgröße, einen Wert für die Umgebungstemperatur und einen Wert für die erste weitere Umgebungsbedingung, optional einen Wert für die zweite weitere Umgebungsbedingung. Die beiden Werte für die beiden Detektionsgrößen sowie der Wert für die Umgebungstemperatur wurden von der Gasmessvorrichtung gemessen, der Wert für die erste weitere Umgebungsbedingung vom Umgebungsbedingungs-Sensor. Bevorzugt beziehen sich diese mindestens vier Werte auf denselben Abtast-Zeitpunkt.

Gemäß dieser Ausgestaltung vermag die Kalibriervorrichtung die beiden funktionalen Zusammenhänge zu erzeugen. Hierfür wendet die Kalibriervorrichtung ein lernendes Verfahren auf die Stichprobe an. In einer Ausgestaltung leitet die Kalibriervorrichtung für jeden Modellparameter jeweils einen Wert her. In einer anderen Ausgestaltung erzeugt die Kalibriervorrichtung abhängig von der Stichprobe ein neuronales Netz.

Weil die Sensor-Anordnung und der Temperatur-Sensor der Gasmessvorrichtung verwendet werden, um die Stichprobe zu erzeugen, wird die Gefahr verringert, dass die beiden verwendeten funktionalen Zusammenhänge für die Gasmessvorrichtung nicht ausreichend genau zutreffend sind. Diese Gefahr wäre größer, wenn eine andere Sensor-Anordnung und / oder ein anderer Temperatur-Sensor verwendet werden würden.

Wie bereits erwähnt, sind in einer Ausgestaltung die beiden funktionale Zusammenhänge zwei analytische Modellgleichungen mit jeweils mindestens einen Modellparameter. Die Kalibriervorrichtung wendet das lernende Verfahren auf die Stichprobe an, um für jeden Modellparameter jeweils einen Wert zu berechnen - es sei denn, für einen Modellparameter wird ein vorgegebener Standardwert verwendet. In einer anderen Ausgestaltung trainiert die Kalibriervorrichtung ein neuronales Netz unter Verwendung der Stichprobe. Wie oben bereits dargelegt, lassen sich diese beiden Ausgestaltungen auch miteinander kombinieren, beispielsweise indem die Kalibriervorrichtung für den oder jeden Modellparameter des einen funktionalen Zusammenhangs jeweils einen Wert berechnet und ein neuronales Netz trainiert, welches den anderen funktionalen Zusammenhang realisiert.

Die erfindungsgemäße Gasmessvorrichtung kann als ein tragbares Gerät ausgestaltet sein. Ein Benutzer führt dieses tragbare Gerät mit sich, und das tragbare Gerät warnt den Benutzer, wenn in seiner Umgebung ein brennbares Zielgas mit einer Konzentration oberhalb einer Schranke vorhanden ist. Bei dieser Anwendung umfasst die Gasmessvorrichtung bevorzugt eine eigene Spannungsversorgungseinheit. Die Gasmessvorrichtung kann auch als ein stationäres Gerät ausgestaltet und bevorzugt mit einem stationären Spannungsversorgungsnetz verbunden oder verbindbar sein. Möglich ist auch, dass mehrere erfindungsgemäße Gasmessvorrichtungen mit jeweils einer eigenen Spannungsversorgungseinheit über einen größeren Bereich verteilt werden, um diesen größeren Bereich auf mindestens ein Zielgas zu überwachen.

Bevorzugt vermag die erfindungsgemäße Gasmessvorrichtung einen Alarm zu generieren, wenn die ermittelte Zielgas-Konzentration oberhalb einer Konzentrations-Schranke liegt, beispielsweise oberhalb derjenigen Konzentrations-Schranke, die auch die Gültigkeit des zweiten funktionalen Zusammenhangs begrenzt. In einer Realisierungsform umfasst die Gasmessvorrichtung eine eigene Ausgabeeinheit, welche den generierten Alarm in mindestens einer von einem Menschen wahrnehmbaren Form auszugeben vermag, insbesondere visuell und / oder akustisch und / oder haptisch (durch Vibrationen). In einer anderen Ausgestaltung veranlasst die Gasmessvorrichtung bei einer Zielgas-Konzentration oberhalb der Konzentrations-Schranke, dass eine Nachricht mit dem Alarm an einen räumlich entfernten Empfänger übermittelt wird und der räumlich entfernte Empfänger den Alarm in einer von einem Menschen wahrnehmbaren Form ausgibt.

Die erfindungsgemäßen Bestandteile der Gasmessvorrichtung können in einem Gehäuse angeordnet sein. Alternativ kann die Auswerteeinheit auch von den übrigen erfindungsgemäßen Bestandteilen der Gasmessvorrichtung räumlich entfernt angeordnet sein und wenigstens zeitweise mit der Sensor-Anordnung in einer Datenverbindung stehen.

Im Folgenden wird die Erfindung anhand eines Ausführungsbeispiels beschrieben. Hierbei zeigt

Figur 1    schematisch eine erste Ausgestaltung der Gasmessvorrichtung, wobei der Detektor und der Kompensator in einer Wheatstone'schen Messbrücke angeordnet sind;
Figur 2    beispielhaft einen als Pellistor ausgestalteten Detektor;
Figur 3    schematisch eine zweite Ausgestaltung der Gasmessvorrichtung;
Figur 4    schematisch die Kalibriervorrichtung der erfindungsgemäßen Anordnung.

Die erfindungsgemäße Gasmessvorrichtung und das erfindungsgemäße Gasmessverfahren vermögen einen räumlichen Bereich auf das Vorhandensein mindestens eines brennbaren Zielgases zu überwachen und / oder die Konzentration eines brennbaren Zielgases in diesem Bereich wenigstens näherungsweise zu ermitteln. In einer Anwendung vermag die Gasmessvorrichtung bei Vorhandensein von mehreren brennbaren Zielgasen ein Maß für eine Summe der Zielgas-Konzentrationen zu ermitteln. Die Gasmessvorrichtung nutzt ein aus dem Stand der Technik bekanntes Verfahren, um ein Gasgemisch in dem Bereich zu analysieren.

Im Ausführungsbeispiel ist die Gasmessvorrichtung als tragbares Gerät ausgestaltet. Ein Benutzer kann dieses Gerät mit sich führen, während er sich in einem zu überwachenden Bereich aufhält. Die Gasmessvorrichtung kann auch ortsfest angebracht sein. Möglich ist, dass nur die nachfolgend beschriebene Sensorik in dem zu überwachenden Bereich angeordnet ist und eine Auswerteeinheit und eine optionale Ausgabeeinheit an räumlich entfernten Positionen. Dies reduziert die Gefahr, dass die Auswerteeinheit oder die Ausgabeeinheit von Schadgasen im Bereich beschädigt werden. Wenigstens zeitweise ist eine Datenverbindung zwischen der Sensorik einerseits und der Auswerteeinheit und der optionalen Ausgabeeinheit andererseits hergestellt.

Im Inneren eines Gehäuses der Gasmessvorrichtung befindet sich ein Detektor. Durch eine Öffnung des Gehäuses diffundiert ein Gasgemisch aus dem zu überwachenden Bereich in das Innere des Gehäuses oder wird in das Innere gefördert, z.B. von einer Pumpe angesogen.

Der Detektor umfasst einen elektrisch leitenden Draht mit einem heizenden Segment. Das heizende Detektor-Segment ist beispielsweise eine Spule, die ein Segment des Drahts bildet. Das elektrisch leitende Material ist beispielsweise Platin oder Rhodium oder Wolfram oder eine Legierung unter Verwendung von mindestens einem dieser Metalle. An diesen Draht wird eine elektrische Spannung U angelegt, sodass elektrischer Strom durch den Draht fließt. Der fließende Strom erhitzt das heizende Detektor-Segment, und das erhitzte heizende Detektor-Segment gibt Wärmeenergie ab. Die abgegebene Wärmeenergie bewirkt, dass im Inneren des Gehäuses mindestens ein brennbares Zielgas oxidiert wird - natürlich nur dann, wenn der Bereich und damit das Innere brennbares Zielgas enthalten.

**In** einer Anwendung ist Methan ($CH_4$) ein zu detektierendes brennbares Zielgas. Durch die Zufuhr von Wärmeenergie reagiert Methan mit Sauerstoff, und es entstehen Wasser und Kohlendioxid. Aus $CH_4$ und $2*O_2$ wird also $2*H_2O$ und $CO_2$.

Bei der Oxidation des Zielgases wird im Inneren des Gehäuses Wärmeenergie freigesetzt. Diese Wärmeenergie wirkt auf den Detektor ein und vergrößert die Temperatur des von Strom durchflossenen und erhitzten Drahts. Diese Temperatur-Erhöhung korreliert mit der freigesetzten Wärmeenergie und damit mit der Konzentration des Zielgases im Inneren des Gehäuses. Eine Gasmessvorrichtung mit einem solchen Detektor wird manchmal als "Wärmetönungs-Sensor" bezeichnet.

Die Temperatur-Änderung verändert eine Eigenschaft des Detektors, welche mit der Detektor-Temperatur korreliert, beispielsweise den elektrischen Widerstand R des vom Strom durchflossenen Drahts des Detektors. Bei vielen elektrisch leitenden Materialien ist der elektrische Widerstand bekanntlich umso höher, je höher die Temperatur des leitenden Materials ist. Die Gasmessvorrichtung misst mindestens eine messbare Größe, welche von der Eigenschaft und damit von der Detektor-Temperatur beeinflusst wird und welche im Folgenden als "Detektionsgröße" bezeichnet wird. Die Detektionsgröße ist beispielsweise direkt die Temperatur oder eine Größe, die mit dem elektrischen Widerstand R des Drahts korreliert, beispielsweise die am Detektor anliegende elektrische Spannung U oder die Stromstärke I oder die vom Detektor-Draht aufgenommene elektrische Leistung P. Falls eine weitere messbare Größe, die ebenfalls von dem elektrischen Widerstand R abhängt, konstant gehalten wird, so korreliert die gemessene Detektionsgröße mit der gesuchten Konzentration des Zielgases. Falls beispielsweise die Stromstärke I des durch den Detektor fließenden Stroms konstant gehalten wird, so korreliert die am Detektor anliegende elektrische Spannung U mit dem elektrischen Widerstand R des Drahts, der Widerstand R korreliert mit der Temperatur des Drahts, die Temperatur des Drahts korreliert mit der Zielgas-Konzentration, und damit korreliert die gemessene elektrische Spannung U mit der gesuchten Zielgas-Konzentration - bei Vorhandensein von mehreren Zielgasen mit der Kombination (Summe) der Zielgas-Konzentrationen.

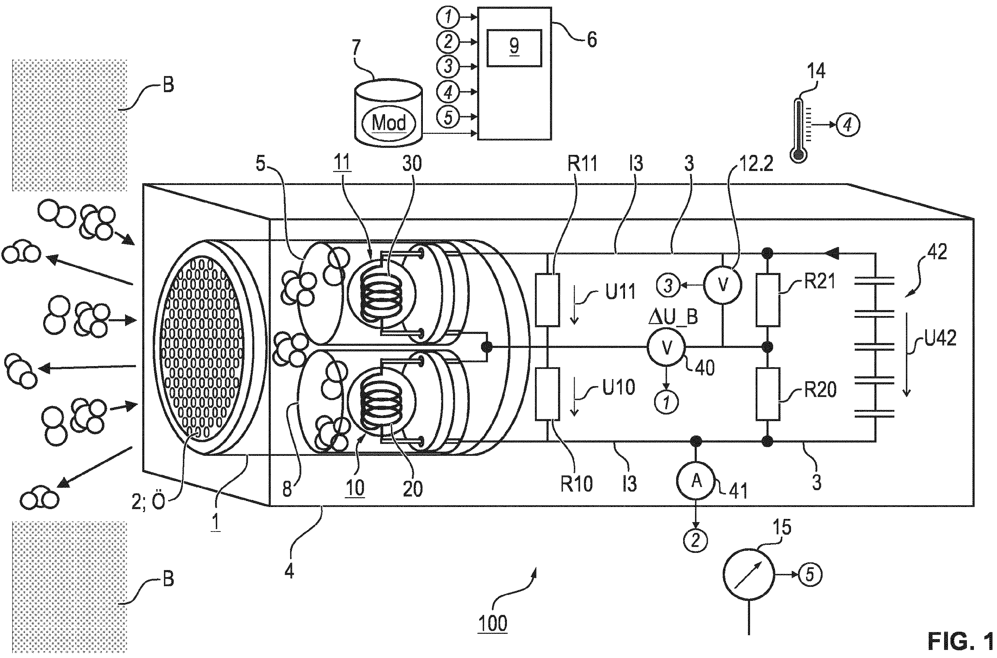

Figur 1 zeigt beispielhaft eine erste Ausgestaltung der erfindungsgemäßen Gasmessvorrichtung 100. Im Ausführungsbeispiel sind ein Detektor 10 in einer Detektorkammer 8 angeordnet und ein Kompensator 11 in einer Kompensatorkammer 5. Die Detektorkammer 8 mit dem Detektor 10 und die Kompensatorkammer 5 mit dem Kompensator 11 befinden sich in einem stabilen Gehäuse 1. Dank einer Öffnung Ö steht das stabile Gehäuse 1 in einer Fluidverbindung mit dem zu überwachenden Bereich, sodass Gas aus dem zu überwachenden Bereich in das Innere des Gehäuses 1 und dort auch in das Innere der Detektorkammer 8 gelangen kann. Ein Flammschutz 2, beispielsweise ein metallisches Gitter, in der Öffnung Ö reduziert das Risiko, dass Flammen aus dem Inneren des stabilen Gehäuses 1 nach außen schlagen. Das stabile Gehäuse 1 ist von einem schematisch gezeigten äußeren Gehäuse 4 umgeben, das sich bevorzugt gut greifen und halten lässt.

Die am Detektor 10 anliegende elektrische Spannung U bewirkt, dass ein elektrischer Strom fließt. Der fließende Strom erhitzt das heizende Detektor-Segment 20 auf eine Arbeitstemperatur, die oft zwischen 400 °C und 500 °C liegt. Diese Arbeitstemperatur allein reicht aber in der Regel nicht aus, um ein brennbares Zielgas im inneren Gehäuse 1 zu oxidieren. Eine höhere Arbeitstemperatur ist oft unerwünscht, weil diese zu einem Verbrennen oder gar zu einem Explodieren von brennbarem Zielgas führen könnte, was oft unerwünscht ist, und außerdem mehr elektrische Energie verbraucht.

Um trotz einer Arbeitstemperatur unterhalb von 500 °C ein brennbares Zielgas oxidieren zu können, umfasst der Detektor 10 ein katalytisches Material, welches in Verbindung mit dem erhitzten heizenden Detektor-Segment 20 das Zielgas oxidiert. Daher wird eine Gasmessvorrichtung mit einem solchen Detektor 10 auch als "katalytischer Sensor" bezeichnet.

In einer häufig verwendeten Realisierung ist das heizende Detektor-Segment 20 von einer elektrischen Isolierung umgeben, beispielsweise von einer Keramikummantelung. Diese elektrische Isolierung isoliert elektrisch das heizende Detektor-Segment 20 und verhindert insbesondere einen unerwünschten Kurzschluss. Die elektrische Isolierung ist thermisch leitend, damit das heizende Detektor-Segment 20 Wärmeenergie in die Umgebung des Detektors 10 abgeben kann und umgekehrt Wärmeenergie aus der Umgebung das heizende Detektor-Segment 20 weiter aufheizen kann. Auf diese elektrische Isolierung ist eine Beschichtung aus einem katalytischen Material aufgetragen. Oder ein katalytisches Material ist in die elektrische Isolierung eingebettet. Diese katalytische Beschichtung kommt in Kontakt mit dem Gasgemisch in dem inneren Gehäuse 1 und damit auch mit einem brennbaren Zielgas. Ein derartig aufgebauter Detektor 10 wird oft als "Pellistor" bezeichnet.

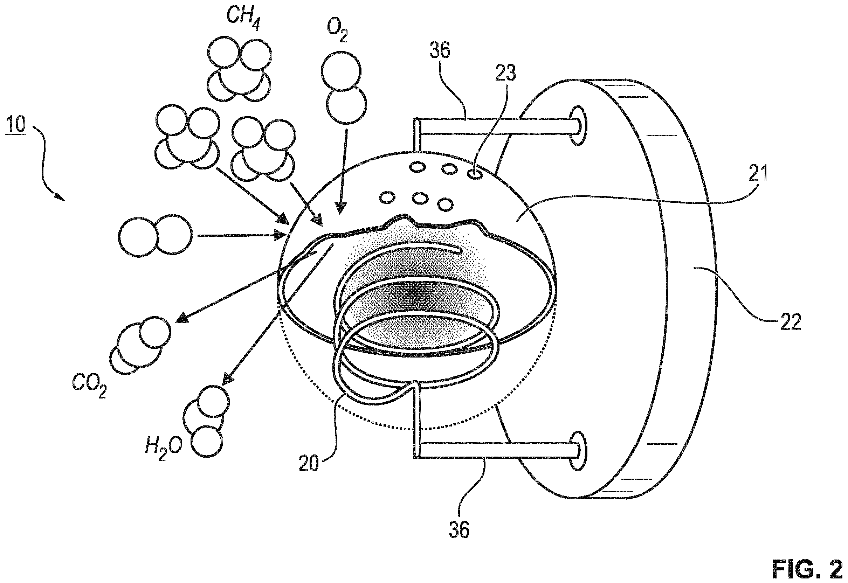

Figur 2 zeigt beispielhaft einen als Pellistor ausgestalteten Detektor 10 sowie schematisch die Umwandlung von Methan ($CH_4$) in $CO_2$ und $H_2O$. Der Detektor 10 umfasst

- einen spiralförmig gewickelten und elektrisch leitenden Draht 20, der als ein heizendes Detektor-Segment fungiert und beispielsweise aus Platin hergestellt ist,
- eine Keramikummantelung 21, welche das heizende Detektor-Segment 20 umgibt und im gezeigten Beispiel die Form einer Vollkugel hat,
- eine katalytische Beschichtung auf der äußeren Oberfläche der Keramikummantelung 21, welche in Figur 2 durch Kreise 23 angedeutet ist,
- eine Montageplatte 22 und
- elektrische Verbindungen und mechanische Halterungen 36 für den Draht 20.

Als katalytisches Material wird beispielsweise Platin oder Palladium verwendet. Alternativ oder zusätzlich zu der katalytischen Beschichtung kann auch katalytisches Material 23 in die Keramikummantelung 21 eingebettet sein.

In einer bevorzugten Ausgestaltung hat die Vollkugel des Detektors 10 eine poröse Oberfläche mit einer katalytischen Beschichtung 23 In einer Ausgestaltung wird diese poröse Oberfläche wie folgt hergestellt: Der Detektor 10 mit der porösen Oberfläche, aber ohne die katalytische Beschichtung wird bereitgestellt. Die katalytische Beschichtung 23 wird auf die poröse Oberfläche aufgetragen, und ein Teil des katalytischen Materials dringt in das Innere des Detektors 10 ein. Dank dieser porösen Oberfläche hat der Detektor 10 eine größere Oberfläche verglichen mit einer glatten Oberfläche. Dank dieser größeren Oberfläche vermag der Detektor 10 besser brennbares Zielgas zu oxidieren, insbesondere weil eine größere Menge von Zielgas in Kontakt mit dem katalytischen Material kommt. Ein Gas kann dank der porösen Oberfläche in tiefere Schichten des Detektors 10 gelangen.

Die Temperatur des Detektors 10 und damit auch die oder eine Detektionsgröße wird aber nicht nur von der freigesetzten Wärmeenergie beeinflusst, sondern auch von Umgebungsbedingungen in dem zu überwachenden Bereich, insbesondere von der Umgebungstemperatur, außerdem von der Luftfeuchtigkeit, dem Umgebungsdruck und von nicht brennbaren Gasen, z.B. $CO_2$, in der Luft. Diese Umgebungsbedingungen können auch die Bedingungen im Inneren des inneren Gehäuses 1 verändern. Diese Umgebungsbedingungen können nämlich ebenfalls die Detektor-Temperatur und damit eine Detektionsgröße beeinflussen, beispielsweise weil die Wärmeleitfähigkeit in der Umgebung des Detektors 10 verändert wird. Gewünscht wird, dass die Gasmessvorrichtung 100 trotz variierender Umgebungsbedingungen einerseits zuverlässig ein brennbares Zielgas zu detektieren vermag und andererseits nur wenige Fehlalarme generiert, also nur selten entscheidet, dass ein Zielgas vorhanden ist, obwohl in Wirklichkeit kein Zielgas oberhalb einer Nachweisgrenze aufgetreten ist, was ein fehlerhaftes Ergebnis ist.

Daher kompensiert die Gasmessvorrichtung 100 konstruktiv und / oder rechnerisch bis zu einem gewissen Grad den Einfluss von Umgebungsbedingungen auf die Detektionsgröße, die von der Temperatur des heizenden Detektor-Segments 20 abhängt. Zu diesem Zweck umfasst die Gasmessvorrichtung 100 zusätzlich zum Detektor 10 einen Kompensator 11, vgl. Figur 1. Der Kompensator 11 umfasst ebenfalls einen Draht mit einem heizenden Kompensator-Segment. Auch an den Kompensator 11 wird eine elektrische Spannung U angelegt, sodass elektrischer Strom fließt und das heizende Segment des Kompensator-Drahts ebenfalls erhitzt wird. Der Kompensator 11 ist ebenfalls den variierenden Umgebungsbedingungen ausgesetzt.

In einer Realisierungsform umfasst der Kompensator 11 ebenfalls einen spiralförmig gewickelten und elektrisch leitenden Draht, der als heizendes Kompensator-Segment fungiert und mit dem Bezugszeichen 30 bezeichnet ist. Der Kompensator 11 umfasst ebenfalls eine Keramikummantelung, eine Montageplatte, elektrische Verbindungen und mechanische Halterungen. Im Gegensatz zum Detektor 10 ist die Keramikummantelung des Kompensators 11 aber nicht mit einer katalytischen Beschichtung versehen.

In einer anderen Realisierungsform ist der Kompensator 11 genauso aufgebaut wie der Detektor 10, umfasst also ebenfalls eine keramische Beschichtung. Jedoch ist die Gasmessvorrichtung 100 so ausgestaltet, dass in einer Zeiteinheit weniger Gas aus dem zu überwachenden Bereich B zu dem Kompensator 11 als zu dem Detektor 10 gelangen kann.

Figur 1 zeigt den Kompensator 11 in der Kompensatorkammer 5. Zu sehen ist, dass der Detektor 10 das heizende Detektor-Segment 20 und der Kompensator 11 ein heizendes Kompensator-Segment 30 umfasst. Im gezeigten Beispiel ist der Kompensator 11 ebenfalls als kugelförmiger Pellistor ausgestaltet, umfasst aber im Gegensatz zum Detektor 10 keine katalytisch aktive Beschichtung 23.

In Figur 1 sind folgende weiteren Bestandteile der Gasmessvorrichtung 100 zu sehen:

- eine eigene Spannungsquelle 42, beispielsweise ein Satz von wiederaufladbaren Akkumulatoren,
- eine elektrische Leitung 3, welche die Spannungsquelle 42 mit dem Detektor 10 und dem Kompensator 11 verbindet,
- einen Spannungs-Sensor 40,
- einen Spannungs-Sensor 12.2,
- einen Stromstärken-Sensor 41,
- zwei elektrische Widerstände R20 und R21 und
- ein signalverarbeitendes Steuergerät 6.

Optional trennt eine nicht gezeigte thermische Barriere im Inneren der Gasmessvorrichtung 100 den Detektor 10 thermisch von dem Kompensator 11. Die Erfindung kann auch ohne eine solche thermische Barriere realisiert werden.

Die Gasmessvorrichtung 100 gemäß Figur 1 ist als Wheatstone'sche Messbrücke aufgebaut. Der Spannungs-Sensor 40 misst die Brückenspannung $\Delta U\_B$ in der Wheatstone'sche Messbrücke. Diese Brückenspannung $\Delta U\_B$ fungiert als die erste Detektionsgröße der Ausgestaltung gemäß Figur 1. Die erste Detektionsgröße hängt bei der ersten Ausgestaltung von der am Detektor 10 anliegenden Spannung U10 und von der am Kompensator 11 anliegenden Spannung U11 wie folgt ab: Sie ist umso größer, je größer die Detektor-Spannung U10 ist, und umso kleiner, je größer die Kompensator-Spannung U11 ist. Der Spannungs-Sensor 12.2 misst die elektrische Spannung U11, die am Kompensator 11 anliegt. Die am Kompensator 11 anliegende elektrische Spannung U11 fungiert als die zweite Detektionsgröße der Ausgestaltung gemäß Figur 1. Der Stromstärken-Sensor 41 misst die Stärke I3 des Stroms, der durch die Leitung 3 fließt.

Der elektrische Widerstand R20 ist parallel zum Detektor 10 geschaltet, der elektrische Widerstand R21 parallel zum Kompensator 11. In Figur 1 sind

- der elektrische Widerstand R10 des Detektors 10,
- der elektrische Widerstand R11 des Kompensators 11,
- die Spannung U42 der Spannungsquelle 42,
- die am Detektor 10 anliegende elektrische Spannung U10 und
- die am Kompensator 11 anliegende elektrische Spannung U11

angedeutet.

Anmerkung: Der Begriff "elektrische Widerstand" bezeichnet zum einen eine elektrische Eigenschaft eines Bauteils, hier beispielsweise den elektrische Widerstand R10 des Detektors 10, und andererseits ein elektrisches Bauteil, beispielsweise den elektrischen Widerstand R20 parallel zum Detektor 10.

Außerdem messen zwei Sensoren 14, 15 der Gasmessvorrichtung 100 zwei unterschiedliche Umgebungsbedingungen. Der Temperatur-Sensor 14 misst die Lufttemperatur in der Umgebung der Gasmessvorrichtung 100, der Druck-Sensor 15 den Luftdruck in der Umgebung. Im Ausführungsbeispiel umfasst die Gasmessvorrichtung 100 keinen Feuchte-Sensor, der die Umgebungsfeuchte misst. Die Umgebungsfeuchte fungiert als oder gehört zur ersten weiteren Umgebungsbedingung im Sinne der Patentansprüche, die nicht direkt gemessen wird, der Umgebungsdruck als eine direkt gemessene zweite weitere Umgebungsbedingung. Möglich ist auch dass der summierte Einfluss der Umgebungsfeuchte und der CO2-Konzentration in der Umgebung als die erste weitere Umgebungsbedingung verwendet wird.

Die Messwerte von den Sensoren 40, 12.2, 41, 14, 15 werden an das Steuergerät 6 übermittelt und vom Steuergerät 6 verarbeitet.

Wie bereits dargelegt, wird die Temperatur des heizenden Detektor-Segments 20 einerseits von der Wärmeenergie beeinflusst, welche bei der Oxidation von brennbarem Zielgas in der Detektorkammer 8 freigesetzt wird. Andererseits wird diese Temperatur von Umgebungsbedingungen beeinflusst. Weil der Kompensator 11 kein brennbares Zielgas oxidiert, wird dessen Temperatur im Wesentlichen von den Umgebungsbedingungen beeinflusst. Außerdem kann die Kompensator-Temperatur dadurch beeinflusst werden, dass viele brennbare Zielgase eine höhere Wärmeleitfähigkeit als Umgebungsluft aufweisen. Die Differenz zwischen den Temperaturen des Detektors 10 und des Kompensators 11 hängt im Wesentlichen nur von der gesuchten Zielgas-Konzentration ab.

In dem in Figur 1 gezeigten Beispiel bilden die Bauteile eine Wheatstone'sche Messbrücke. Der Detektor 10 und der Kompensator 11 sind in Reihe geschaltet. Der elektrische Widerstand des Spannungs-Sensors 40 ist hoch im Vergleich zu den elektrischen Widerständen der Bauteile 10, 11, R20, R21. In einer Ausgestaltung misst der Spannungs-Sensor 40 direkt die sogenannte Brückenspannung $\Delta U\_B = (U10 - U11) / 2$. Die korrigierte Brückenspannung $\Delta U\_B_{korr} = \Delta U\_B - \Delta U\_B0$ korreliert mit der gesuchten Zielgas-Konzentration. Hierbei ist $\Delta U\_B0$ der Nullpunkt, also diejenige Brückenspannung $\Delta U\_B$, die dann auftritt, wenn im zu überwachenden Bereich und damit im Inneren der Gasmess-

**EP 4 321 859 B1**

vorrichtung 100 kein brennbares Zielgas vorhanden ist. Der Spannungs-Sensor 12.2 misst die Spannung U11, die am Kompensator 11 anliegt.

Bekanntlich korreliert die Temperatur eines elektrisch leitenden Bauteils mit dessen elektrischen Widerstand. In einer Ausgestaltung wird die Stromstärke I3 durch eine automatische Regelung (closed-loop control) konstant gehalten. Genauer gesagt: Eine Regelung wird mit dem Regelungsziel durchgeführt, die Stromstärke I3 konstant zu halten. Das Steuergerät 6 führt diese Regelung durch. Dank dieser Regelung korreliert die Brückenspannung $\Delta U\_B$ mit dem elektrischen Widerstand R des Detektors 10 und ist daher ein Maß für die Temperatur des Detektors 10.

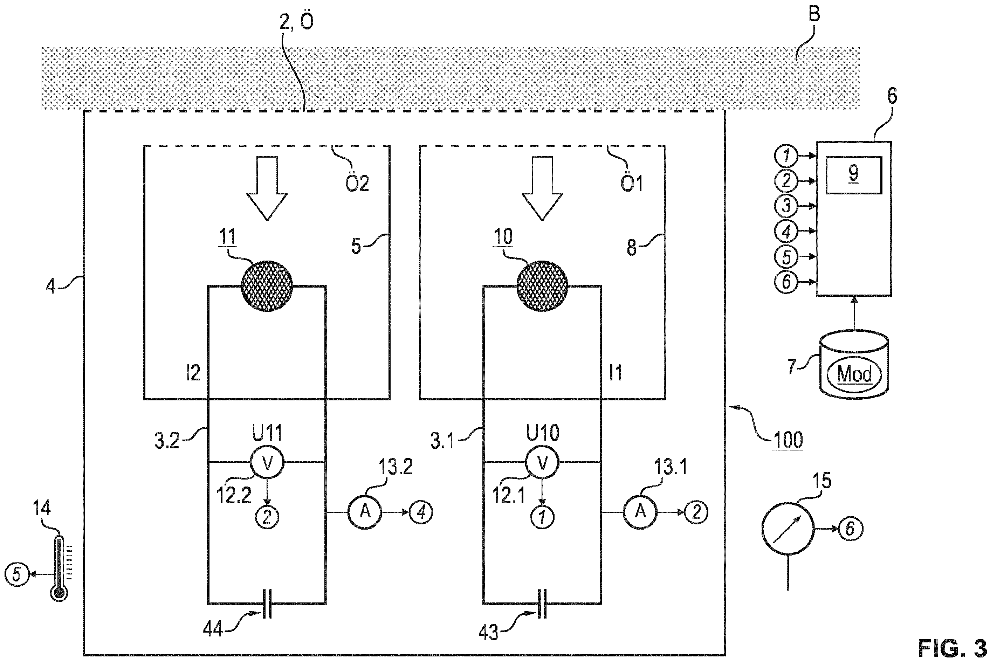

Figur 3 zeigt eine zweite Ausführungsform der Gasmessvorrichtung 100. Gleiche Bezugszeichen haben die gleiche Bedeutung wie in Figur 1. Die Detektorkammer 8 steht über eine Öffnung Ö1 in einer Fluidverbindung mit dem zu überwachenden Bereich B, die Kompensatorkammer 5 über eine Öffnung Ö2.

Gemäß der zweiten Ausgestaltung werden der Detektor 10 und der Kompensator 11 unabhängig voneinander mit elektrischer Energie versorgt. Ein erster elektrischer Stromkreis verbindet den Detektor 10 mit einer ersten Spannungsquelle 43, ein zweiter elektrischer Stromkreis den Kompensator 11 mit einer zweiten Spannungsquelle 44.

Ein Spannungs-Sensor 12.1 misst die elektrische Spannung U10, die am Detektor 10 anliegt. Die elektrische Spannung U10 ist in einer Realisierungsform der zweiten Ausgestaltung die erste Detektionsgröße. Ein Stromstärken-Sensor 13.1 misst die Stärke I.1 des elektrischen Stroms, der durch den Stromkreis für den Detektor 10 fließt. Ein Spannungs-Sensor 12.2 misst die elektrische Spannung U11, die am Kompensator 11 anliegt. Die am Kompensator 11 anliegende Spannung U11 fungiert bei der zweiten Ausgestaltung als die zweite Detektionsgröße. Ein Stromstärken-Sensor 13.2 misst die Stärke I.2 des elektrischen Stroms, der durch den Stromkreis für den Kompensator 11 fließt.

Berechnet und in einer alternativen Realisierungsform als erste Detektionsgröße verwendet wird die Spannungs-Differenz $\Delta U$ = U10 - U11, die idealerweise gleich Null ist, falls kein brennbares Zielgas vorhanden ist, in der Praxis aber auch bei Abwesenheit von brennbarem Zielgas von null differiert. Daher wird eine korrigierte Spannungs-Differenz $\Delta U_{korr}$ = U10 - U11 - $\Delta$U0 berechnet. Gemäß einer Realisierungsform der zweiten Ausgestaltung korreliert die korrigierte Spannungs-Differenz $\Delta U_{korr}$ = U10 - U11 - $\Delta$U0 mit der Zielgas-Konzentration. Der Nullpunkt $\Delta$U0 ist die Spannungs-Differenz U10 - U11, die dann auftritt, wenn kein brennbares Zielgas vorhanden ist. Die korrigierte Spannungs-Differenz $\Delta U_{korr}$ = U10 - U11 - $\Delta$U0 fungiert in dieser Realisierungsform als die erste Detektionsgröße. Allgemeiner: Bei der alternativen Realisierungsform ist die erste Detektionsgröße umso größer, je größer U10 ist, und umso kleiner, je größer U11 ist.

Die Detektionsgröße elektrische Spannung U wird von der gesuchten Zielgas-Konzentration sowie im Ausführungsbeispiel von den drei Umgebungsbedingungen Umgebungstemperatur, Umgebungsfeuchte und Umgebungsdruck beeinflusst. In vielen Fällen kann jeder dieser vier Einflüsse als linear angenommen werden. Dann gilt:

$$(1) \qquad U10 = k_{10}*con + m_{Temp,10}*Temp + m_{P,10}*P + m_{Hum,10}*Hum + U10,0$$

und

$$(2) \qquad U11 = k_{11}*con + m_{Temp,11}*Temp + m_{P,11}*P + m_{Hum,11}*Hum + U11,0$$

und daher

$$U10 - U11 = (k_{10} - k_{11})*con + (M_{Temp,10} - M_{Temp,11})*Temp + (M_{P,10} - M_{P,11})*P + (m_{Hum,10} - m_{Hum,11})*Hum + (U10,0 - U11,0) \qquad (3)$$

und

$$(4) \qquad \Delta U\_B_{korr} = \Delta U\_B - \Delta U\_B0 \text{ mit } \Delta U\_B = (U10 - U11)/2$$

und

$$\Delta U\_B = (k_{10} - k_{11})/2*con + (M_{Temp,10} - m_{Temp,11})/2*Temp + (m_{P,10} - m_{P,11})/2*P + (M_{Hum,10} - m_{Hum,11})/2*Hum + \Delta U\_B0. \qquad (5)$$

Die Symbole haben die folgenden Bedeutungen:

| con | gesuchte Zielgas-Konzentration |
| --- | --- |

| Temp | Umgebungstemperatur |
|---|---|
| P | Umgebungsdruck |
| Hum | Umgebungsfeuchte (humidity) |
| $k_{10}$ | Proportionalitätsfaktor für die Abhängigkeit der Detektor-Spannung 10 von der Zielgas-Konzentration con |
| $k_{11}$ | Proportionalitätsfaktor für die Abhängigkeit der Kompensator-Spannung 11 von der Zielgas-Konzentration con |
| $m_{Temp,10}$, $m_{Temp,11}$ | Proportionalitätsfaktoren für die Umgebungstemperatur Temp |
| $m_{P,10}$, $m_{P,11}$ | Proportionalitätsfaktoren für den Umgebungsdruck P |
| MHum,10, MHum,11 | Proportionalitätsfaktoren für die Umgebungsfeuchte Hum |
| U10 | am Detektor 10 anliegende elektrische Spannung |
| U11 | am Kompensator 11 anliegende elektrische Spannung |
| U10,0 | Nullpunkt der am Detektor 10 anliegenden elektrischen Spannung, also die elektrische Spannung, die bei einem Zustand frei von brennbarem Zielgas anliegt |
| U11,0 | Nullpunkt der am Kompensator 11 anliegenden elektrischen Spannung, also die elektrische Spannung, die bei einem Zustand frei von brennbarem Zielgas anliegt |
| $\Delta U\_B$ | Brückenspannung, wird vom Spannungs-Sensor 40 gemessen, gleich (U10 - U11) / 2 |
| $\Delta U\_B0$ | Nullpunkt der Brückenspannung $\Delta U\_B$ |

In einer Ausgestaltung vermag der Kompensator 11 überhaupt kein brennbares Zielgas zu oxidieren, und der Faktor $k_{11}$ ist Null. Möglich ist auch, dass der Faktor $k_{11}$ kleiner als Null ist. Der Grund: Viele zu detektierende brennbare Zielgase haben eine höhere Wärmeleitfähigkeit als Umgebungsluft, sodass sie den Kompensator 11 kühlen, wodurch die elektrische Spannung U11 geringer ist als bei Abwesenheit von brennbarem Zielgas. In einer anderen Ausgestaltung vermag der Kompensator 11 weniger brennbares Zielgas als der Detektor 10 zu oxidieren, und $k_{11}$ ist größer als Null, aber um den Faktor 10, bevorzugt um den Faktor 20, kleiner als $k_{10}$.

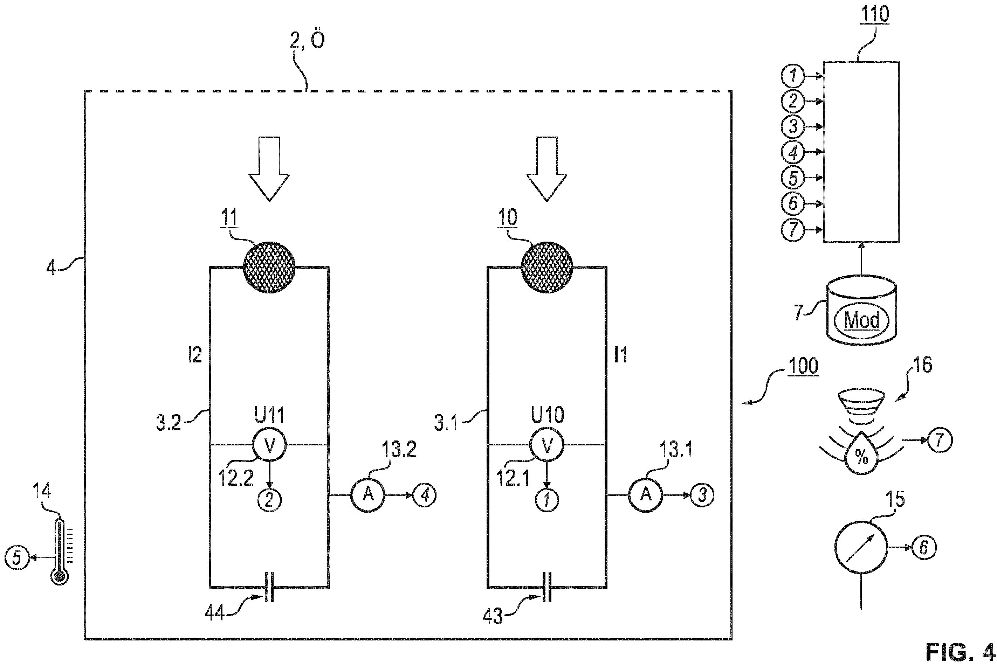

Bevorzugt führt eine Kalibriervorrichtung 110 nacheinander zwei Justierungen durch. Figur 4 zeigt schematisch diese Kalibriervorrichtung 110.

Bei beiden Justierungen werden unterschiedliche Zustände hergestellt. Bei jedem Zustand sind die Konzentration con von brennbarem Zielgas, die Umgebungstemperatur Temp, der Umgebungsdruck P und die Umgebungsfeuchte Hum bekannt. Die Umgebungstemperatur Temp und der Umgebungsdruck P werden in einer Ausgestaltung durch den Temperatur-Sensor 14 bzw. den Druck-Sensor 15 der Gasmessvorrichtung 100 gemessen, die Umgebungsfeuchte Hum durch einen Feuchte-Sensor 16, der im Ausführungsbeispiel kein Bestandteil der Gasmessvorrichtung 100 ist und nur für die Justierung benutzt wird.

In diesem Zustand mit bekannten Umgebungsbedingungen werden die Brückenspannung $\Delta U\_B$ und die Kompensator-Spannung U11 (erste Ausgestaltung gemäß Figur 1) bzw. die Detektor-Spannung U10 und die Kompensator-Spannung U11 (zweite Ausgestaltung gemäß Figur 3) gemessen. Aus der Brückenspannung $\Delta U\_B$ und der Kompensator-Spannung U11 wird bei der ersten Ausgestaltung die Detektor-Spannung U10 hergeleitet. Die unterschiedlichen Zustände liefern jeweils eine Stichprobe mit mehreren Stichprobenelementen. Die beiden Justierungen liefern also zwei Stichproben.

Bei der ersten Justierung werden nacheinander verschiedene Zustände hergestellt, bei denen kein brennbares Zielgas vorhanden ist. Die Zustände bei der ersten Justierung unterscheiden sich hinsichtlich der Umgebungstemperatur Temp, des Umgebungsdrucks P und / oder der Umgebungsfeuchte Hum voneinander. Die erste Justierung liefert eine erste Stichprobe. Jedes Stichprobenelement umfasst jeweils einen Wert für die Umgebungstemperatur Temp, die Umgebungsdruck P und die Umgebungsfeuchte Hum sowie den resultierenden Wert für die Detektor-Spannung U10 bzw. für die Kompensator-Spannung U11. Bei der ersten Justierung werden Werte für die folgenden Parameter bestimmt: die Nullpunkte U10,0 und U11,0 sowie die Proportionalitätsfaktoren $m_{Temp,10}$, $m_{P,10}$ und $m_{Hum,10}$. und $m_{Temp,11}$, $m_{P,11}$ und $m_{Hum,11}$ Die gesuchten Parameterwerte werden bevorzugt durch eine Regressionsanalyse festgelegt.

Eine alternative Realisierung erfordert keine analytische Modellgleichung mit Modellparametern. Vielmehr wird ein neuronales Netz mithilfe der gerade beschriebenen Stichprobe trainiert.

Bei der zweiten Justierung werden nacheinander Zustände hergestellt, bei denen ein brennbares Zielgas mit unterschiedlichen Konzentrationen in der Umgebung der Gasmessvorrichtung 100 und daher auch in der Detektorkammer 8 und in der Kompensatorkammer 5 vorhanden ist. Auch die Zustände bei der zweiten Justierung weisen bevorzugt unterschiedliche Umgebungsbedingungen auf. Bei der zweiten Justierung werden Werte für die beiden Proportionalitätsfaktoren $k_{10}$ und $k_{11}$ festgelegt. Die Werte für die sechs Proportionalitätsfaktoren $m_{Temp,10}$, $m_{P,10}$ und $m_{Hum,10}$. und $m_{Temp,11}$, $m_{P,11}$ und $m_{Hum,11}$, die bei der ersten Justierung festgelegt wurden, werden bevorzugt bei der zweiten Justierung wieder verwendet.

In aller Regel sind der Detektor 10 und der Kompensator 11 annähernd gleich sensitiv für die Umgebungsfeuchte Hum, sodass $m_{Hum,10} = m_{Hum,11}$ gilt. Dann vereinfacht sich (3) zu

$$U10 - U11 = (k_{10} - k_{11})*con + (M_{Temp,1\,0} - m_{Temp,11})*Temp + (M_{P,10} - M_{P,11})*P + (U10,0 - U11,0) \tag{6}$$

und (5) zu

$$\Delta U\_B = (k_{10} - k_{11})/2*con + (M_{Temp,10} - m_{Temp,11})/2*Temp + (m_{P,10} - m_{P,11})/2*P + \Delta U\_B0. \tag{7}$$

In vielen Fällen sind der Kompensator 10 und der Detektor 11 außerdem gleich sensitiv für die Umgebungstemperatur Temp und den Umgebungsdruck P, sodass die weiter vereinfachten Gleichungen

$$(8) \quad U10 - U11 = (k_{10} - k_{11})*con + (U10,0 - U11,0)$$

und

$$(9) \quad \Delta U\_B = (k_{10} - k_{11})/2*con + \Delta U\_B0$$

verwendbar sind.

Falls der Kompensator 11 überhaupt nicht oder nur in vernachlässigbar geringem Umfange brennbares Zielgas zu oxidieren vermag, so ist der Faktor $k_{11}$ gleich null, sodass sich die oben stehenden Berechnungsvorschriften weiter vereinfachen.

Die Auswerteeinheit 9 hat wenigstens zeitweise Lesezugriff auf einen Datenspeicher 7. In diesem Datenspeicher 7 ist in rechnerauswertbare Form ein Modell Mod abgespeichert. In einer Realisierungsform umfasst das Modell Mod folgende analytischen Modellgleichungen:

- bei der ersten Ausgestaltung gemäß Figur 1 die nach con aufgelösten Gleichungen (7) oder (9) und
- bei der zweiten Ausgestaltung gemäß Figur 3 die nach con aufgelösten Gleichungen (6) oder (8).

Falls in dem zu überwachenden Bereich kein brennbares Zielgas oberhalb einer Nachweisgrenze vorhanden ist, so gilt con = 0. Die Gleichungen (1), (2), (3) und (5) vereinfachen sich zu

$$(10) \quad U10 = m_{Temp,10}*Temp + m_{P,10}*P + m_{Hum,10}*Hum + U10,0$$

und

$$(11) \quad U11 = m_{Temp,11}*Temp + m_{P,11}*P + m_{Hum,11}*Hum + U11,0$$

und (12) $\Delta U\_B = (M_{Temp,10} - m_{Temp,11})/2*Temp + (m_{P,10} - m_{P,11})/2*P + (M_{Hum,10} - m_{Hum,11})/2*Hum + \Delta U\_B0.$

Aus (10) und (11) folgt außerdem:

$$U10 + U11 = (M_{Temp,10} + m_{Temp,11})*Temp + (M_{P,10} + m_{P,11})*P + (m_{Hum,10} + m_{Hum,11})*Hum + (U10,0 + U11,0). \tag{13}$$

Das im Datenspeicher 7 abgespeicherten Modell Mod umfasst im Ausführungsbeispiel zusätzlich folgende analytischen Modellgleichungen:

- bei der ersten Ausgestaltung gemäß Figur 1 die nach Hum aufgelöste Gleichung (12) und
- bei der zweiten Ausgestaltung gemäß Figur 2 mindestens eine der nach Hum aufgelösten Gleichungen (10), (11) und (13).

Möglich ist, die erste Justierung und / oder die zweite Justierung nach einem Einsatz der Gasmessvorrichtung 100 erneut durchzuführen, beispielsweise wenn die bisherige Einsatzdauer der Gasmessvorrichtung 100 eine vorgegebene Zeitdauer-Schranke überstiegen hat und / oder wenn die bisherige Belastung der Gasmessvorrichtung 100 mit brennbarem Zielgas eine Zielgas-Schranke überstiegen hat. In einer Ausgestaltung werden bei der zweiten Justierung die gleichen Schritte wie bei der ersten Justierung durchgeführt. Möglich ist auch, dass die Nullwerte häufiger bestimmt werden als die Modellparameter.

In einer anderen Ausgestaltung wird bei der zweiten Justierung ein vereinfachter Ablauf durchgeführt. Lediglich die Faktoren $k_{10}$ und $k_{11}$ sowie die Nullpunkt-Spannungen U10,0, U11,0 und / oder $\Delta U\_B0$ werden erneut bestimmt. Möglich ist, dass bei den Umgebungsbedingungen, die bei der zweiten Justierung vorliegen, die Nullpunkt-Spannungen U10,0, U11,0 und / oder $\Delta U\_B0$ erneut bestimmt werden. Hingegen wird davon ausgegangen, dass die Faktoren $m_{Temp,10}$, $m_{Temp,11}$, $m_{P,10}$, $m_{P,11}$, $m_{Hum,10}$ und $m_{Hum,11}$ während der gesamten Einsatzdauer der Gasmessvorrichtung 100 konstant bleiben und daher nicht erneut bestimmt werden müssen. Vielmehr werden die Werte, die bei der ersten Justierung bestimmt wurden, weiterverwendet.

In einer Realisierungsform wird bei der zweiten Justierung die Gasmessvorrichtung 100 einerseits einer Umgebung mit Atemluft, die frei von brennbarem Zielgas ist, und andererseits einer Umgebung mit einem Gasgemisch umfassend Atemluft und ein brennbares Zielgas ausgesetzt, wobei dieses Gasgemisch bevorzugt aus einer Flasche stammt und daher keine nennenswerte Feuchtigkeit aufweist.

Nachfolgend wird beschrieben, wie die Gasmessvorrichtung 100 sowohl dafür verwendet wird, um einerseits das Vorhandensein von brennbarem Zielgas zu detektieren und / oder die Zielgas-Konzentration con zu messen und um andererseits die Umgebungsfeuchte Hum näherungsweise zu messen.

Falls brennbares Zielgas in den zu überwachenden Bereich B vorhanden ist, so werden die Brückenspannung $\Delta U\_B$ (erste Ausgestaltung) bzw. die Detektor-Spannung U10 oder die Spannungs-Differenz $\Delta U = U10 - U11$ (zweite Ausgestaltung) im Wesentlichen von der gesuchten Zielgas-Konzentration con beeinflusst. Der Kompensator 11 kompensiert weitgehend den Einfluss der Umgebungstemperatur Temp, des Umgebungsdrucks P und der Umgebungsfeuchte Hum. Die gesuchte Zielgas-Konzentration con wird daher in einer Realisierungsform abhängig von der gemessenen Brückenspannung $\Delta U\_B$ bzw. der Spannungs-Differenz $\Delta U$ sowie von den beiden Proportionalitätsfaktoren $k_{10}$ und $k_{11}$ ermittelt, bevorzugt gemäß einer der nach con aufgelösten Modellgleichungen (6) oder (7), die im Datenspeicher 7 abgespeichert sind. Möglich ist auch, zusätzlich den Einfluss der Umgebungstemperatur Temp und des Umgebungsdrucks P zu berücksichtigen und daher bevorzugt eine der nach con aufgelösten abgespeicherten Modellgleichungen (8) oder (9) zu verwenden.

Falls die korrigierte Brückenspannung $\Delta U\_B_{korr}$ in einem vorgegebenen Bereich liegt, bevorzugt in einem Toleranzband um den Nullpunkt herum, so ist im zu überwachenden Bereich B kein brennbares Zielgas oberhalb der Nachweisgrenze vorhanden. Falls kein brennbares Zielgas vorhanden ist, so werden sowohl die Detektor-Spannung U10 als auch die Kompensator-Spannung U11 von der Umgebungstemperatur Temp, dem Umgebungsdruck P und der Umgebungsfeuchte Hum beeinflusst. Der Temperatur-Sensor 14 misst die Umgebungstemperatur Temp, und der Druck-Sensor 15 misst den Umgebungsdruck P. Durch die erste Justierung sind die Proportionalitätsfaktoren $m_{Temp,11}$, $m_{P,11}$ und $m_{Hum,11}$ festgelegt.

In einer Ausgestaltung wird die am Kompensator 11 anliegende Spannung U11 verwendet, um die Umgebungsfeuchte Hum bei Abwesenheit von brennbarem Zielgas zu ermitteln, bevorzugt gemäß der nach Hum aufgelösten und abgespeicherten Modellgleichung

$$(11) \quad U11 = m_{Temp,11}*Temp + m_{P,11}*P + m_{Hum,11}*Hum + U11,0.$$

Möglich ist auch, die am Detektor 10 anliegende Spannung U10 zu verwenden, um die Umgebungsfeuchte Hum zu ermitteln. Bevorzugt wird hierfür die nach Hum aufgelöste und abgespeicherte Modellgleichung

$$(10) \quad U10 = m_{Temp,10}*Temp + m_{P,10}*P + m_{Hum,10}*Hum + U10,0$$

verwendet. Bei der ersten Ausgestaltung wird die Detektor-Spannung U10 aus der Kompensator-Spannung U11 und der Brückenspannung $\Delta U\_B$ hergeleitet, bei der zweiten Ausgestaltung direkt gemessen.

Möglich ist auch, die Spannungs-Summe U10 + U11 herzuleiten und die nach Hum aufgelöste und abgespeicherte Modellgleichung

$$U10 + U11 = (U10,0 + U11,0) + (m_{Temp,10} + m_{Temp,11})*Temp + (m_{P,10} + m_{P,11})*P + (m_{Hum,10} + m_{Hum,11})*Hum. \quad (13)$$

zu verwenden.

In vielen Fällen ist die Annahme gerechtfertigt, dass der Detektor 10 und der Kompensator 11 tatsächlich gleich empfindlich für die Umgebungstemperatur Temp, die Umgebungsfeuchtigkeit Hum und den Luftdruck P sind. Bei dieser Annahme vereinfacht sich (13) zu

$$U10 + U11 = (U10,0 + U11,0) + 2*m_{Temp,10}*Temp + 2*m_{P,10}*P + 2*m_{Hum,10}*Hum. \qquad (13)$$

In einer Ausgestaltung wird zu jedem Abtast-Zeitpunkt, an dem die Sensoren der Gasmessvorrichtung 100 jeweils einen Messwert liefern, ein Messwerte-Vektor generiert und an einen räumlich entfernten Empfänger übermittelt. Dieser Messwerte-Vektor umfasst die Messwerte der folgenden Größen:

- die Kompensator-Spannung U11 sowie die Brückenspannung $\Delta U\_B$ (erste Ausgestaltung) bzw. die Detektor-Spannung U10 oder die Differenz U10 - U11 (zweite Ausgestaltung) sowie
- der Umgebungstemperatur Temp und des Umgebungsdrucks P.

Falls zum Abtast-Zeitpunkt brennbares Zielgas vorhanden ist, so umfasst der Messwerte Vektor zusätzlich die gemessene Zielgas-Konzentration con. Falls zum Abtast-Zeitpunkt kein brennbares Zielgas vorhanden ist, so umfasst der Messwerte-Vektor zusätzlich den hergeleiteten Wert der Umgebungsfeuchte Hum.

Die übermittelten Messwerte-Vektoren lassen sich beispielsweise für die folgende Anwendung verwenden: Eine Fehlfunktion der Gasmessvorrichtung 100 wurde festgestellt. Gesucht sind solche Zielgas-Konzentrationen und Umgebungsbedingungen, bei denen diese Fehlfunktion aufgetreten ist.

**Bezugszeichenliste**

| 1 | stabiles Gehäuse der Gasmessvorrichtung 100, nimmt den Detektor 10 und den Kompensator 11 auf, weist die Öffnung Ö auf, vom äußeren Gehäuse 4 umgeben |
|---|---|
| 2 | Flammensperre in der Öffnung Ö, beispielsweise als Metallgitter und / oder Sinterplatte ausgestaltet |
| 3 | elektrische Leitung oder Leitungs-Anordnung, welche den Detektor 10 und den Kompensator 11 mit der Spannungsquelle 42 verbindet und dadurch mit elektrischer Energie versorgt |
| 4 | äußeres Gehäuse der Gasmessvorrichtung 100, nimmt das innere Gehäuse 1, die optionalen elektrischen Widerstände R20 und R21, die Sensoren für Strom, Spannung und Temperatur, das Steuergerät 6 mit der Auswerteeinheit 9 und die Spannungsquelle 42 auf, weist die Öffnung Ö auf |
| 5 | Kompensatorkammer im Gehäuse 1, umgibt den Kompensator 11, weist die Öffnung Ö2 auf |
| 6 | signalverarbeitendes Steuergerät, empfängt Signale von den Sensoren 12.1, 12.2, 13.1, 13.2, 40 und 41, steuert bei Bedarf die elektrischen Widerstände R10 und R11 an, umfasst die Auswerteeinheit 9 |
| 7 | Datenspeicher, in dem das Modell Mod abgespeichert ist |
| 8 | Detektorkammer im Gehäuse 1, umgibt den Detektor 10, weist die Öffnung Ö1 auf |
| 9 | signalverarbeitende Auswerteeinheit, empfängt Messwerte von den Sensoren und wertet diese aus |
| 10 | Detektor, in der Detektorkammer 8 angeordnet, umfasst das heizende Detektor-Segment 20, die Keramikummantelung 21, eine Beschichtung 23 oder Einbettung aus einem katalytischen Material und die Montageplatte 22, ist als Pellistor ausgestaltet |
| 11 | Kompensator, umfasst das heizende Kompensator-Segment 30, in der Kompensatorkammer 5 angeordnet |
| 12.1 | Spannungs-Sensor, misst die am Detektor 10 anliegende elektrische Spannung U10 |
| 12.2 | Spannungs-Sensor, misst die am Kompensator 11 anliegende elektrische Spannung U11 |
| 13.1 | Stromstärken-Sensor, misst die Stärke I.1 des durch den Detektor 10 fließenden elektrischen Stroms |

(fortgesetzt)

| | |
|---|---|
| 13.2 | Stromstärken-Sensor, misst die Stärke I.2 des durch den Kompensator 11 fließenden elektrischen Stroms |
| 14 | Temperatur-Sensor, misst die Temperatur Temp in der Umgebung der Gasmessvorrichtung 100 |
| 15 | Druck-Sensor, misst den Luftdruck P in der Umgebung der Gasmessvorrichtung 100 |
| 16 | Feuchte-Sensor, misst ein Maß für die Umgebungsfeuchte Hum |
| 20 | spiralförmiger elektrisch leitender Draht, fungiert als das heizende Detektor-Segment |
| 21 | Keramikummantelung um den Draht 20, mit einer katalytischen Beschichtung 23 versehen |
| 22 | Montageplatte, welche den Draht 20 und die Keramikummantelung 21 hält |
| 23 | Beschichtung der Keramikummantelung 21 aus einem katalytischen Material |
| 30 | heizendes Kompensator-Segment |
| 36 | mechanische Halterungen für den Draht 20 |
| 40 | Spannungs-Sensor, misst die elektrische Brücken-Spannung, nämlich die Hälfte der Spannungs-Differenz $\Delta U = U10 - U11$ |
| 41 | Stromstärken-Sensor, misst die Stromstärke I3 in der Leitung 3 |
| 42 | Spannungsversorgungseinheit |
| 100 | erfindungsgemäße Gasmessvorrichtung |
| 110 | Kalibriervorrichtung, umfasst den Feuchte-Sensor 16, erzeugt das Modell Mod, das in Datenspeicher 7 abgespeichert wird |
| B | zu überwachender Bereich |
| con | gesuchte Zielgas-Konzentration |
| I3 | übereinstimmende Stärke des elektrischen Stroms, der durch den Detektor 10 und den Kompensator 11 fließt (Wheatstone'sche Messbrücke) |
| I1 | Stärke des elektrischen Stroms, welcher durch den Detektor 10 fließt |
| I2 | Stärke des elektrischen Stroms, welcher durch den Kompensator 11 fließt |
| $k_{10}$ | Proportionalitätsfaktor für die Abhängigkeit der Detektor-Spannung 10 von der Zielgas-Konzentration |
| $k_{11}$ | Proportionalitätsfaktor für die Abhängigkeit der Kompensator-Spannung 11 von der Zielgas-Konzentration |
| MHum,10, MHum,11 | Proportionalitätsfaktoren für die Umgebungsfeuchte Hum |
| $m_{P,10}$, $m_{P,11}$ | Proportionalitätsfaktoren für den Umgebungsdruck P |
| $m_{Temp,10}$, $m_{Temp,11}$ | Proportionalitätsfaktoren für die Umgebungstemperatur Temp |
| Mod | Modell, das in Datenspeicher 7 abgespeichert ist und mindestens einen Zusammenhang zwischen elektrischer Spannung, Zielgas-Konzentration und Umgebungsbedingungen beschreibt, besteht in einer Ausgestaltung aus mehreren Modellgleichungen |
| Ö | Öffnung im Gehäuse 1, durch welches ein Gasgemisch aus dem Bereich B in das Innere des Gehäuses 1 fließen kann und in welche der Flammschutz 2 eingesetzt ist |
| Ö1 | Öffnung in der Detektorkammer 8 |
| Ö2 | Öffnung in der Kompensatorkammer 5 |
| R10 | elektrischer Widerstand des Detektors 10, korreliert mit der Temperatur des Detektors 10 |
| R11 | elektrischer Widerstand des Kompensators 11, korreliert mit der Temperatur des Kompensators 11 |
| R20 | als elektrischer Widerstand ausgestaltetes Bauteil, das parallel zum Detektor 10 geschaltet ist |

(fortgesetzt)

| R21 | als elektrischer Widerstand ausgestaltetes Bauteil, das parallel zum Kompensators 11 geschaltet ist |
|---|---|
| U10 | elektrische Spannung, die am Detektor 10 anliegt |
| U10,0 | Nullpunkt der am Detektor 10 anliegenden elektrischen Spannung |
| U11 | elektrische Spannung, die am Kompensator 11 anliegt |
| U11,0 | Nullpunkt der am Kompensator 11 anliegenden elektrischen Spannung |
| $\Delta U$ | Spannungs-Differenz, gleich U10 - U11 |
| $\Delta U0$ | Nullpunkt für die Spannungs-Differenz |
| $\Delta U\_B$ | Brückenspannung, gleich (U10 - U11) / 2 |
| $\Delta U\_B0$ | Nullpunkt für die Brückenspannung $\Delta U\_B$, gleich (U10,0 - U11,0) / 2 |
| $\Delta U\_B_{korr}$ | korrigierte Brückenspannung, gleich $\Delta U\_B - \Delta U0\_B$ |
| U42 | elektrische Spannung der Spannungsquelle 42 |

**Patentansprüche**

1. Gasmessvorrichtung (100) zum Messen der Konzentration eines brennbaren Zielgases ($CH_4$),

   wobei die Gasmessvorrichtung (100)

   - einen Detektor (10) mit einem heizenden Detektor-Segment (20),
   - einen Kompensator (11) mit einem heizenden Kompensator-Segment (30),
   - eine Sensor-Anordnung (12.1, 12.2, 13.1, 13.2, 40, 41),
   - einen Temperatur-Sensor (14),
   - eine signalverarbeitende Auswerteeinheit (9) und
   - einen Datenspeicher (7), auf den die Auswerteeinheit (9) wenigstens zeitweise Lesezugriff hat,

   umfasst,
   wobei die Gasmessvorrichtung (100) dazu ausgestaltet ist,

   - eine elektrische Spannung (U10) an den Detektor (10) anzulegen, sodass ein elektrischer Strom (I3, 11) durch das heizende Detektor-Segment (20) fließt und das heizende Detektor-Segment (20) erhitzt wird, und
   - eine elektrische Spannung (U11) an den Kompensator (11) anzulegen, sodass ein elektrischer Strom (I3, I2) durch das heizende Kompensator-Segment (30) fließt und das heizende Kompensator-Segment (30) erhitzt wird,

   wobei der Detektor (10) dazu ausgestaltet ist, durch eine Erhitzung des heizenden Detektor-Segments (20) im Inneren der Gasmessvorrichtung (100) befindliches brennbares Zielgas ($CH_4$) zu oxidieren,
   wobei

   - der Kompensator (11) in geringerem Umfang als der Detektor (10) oder sogar überhaupt nicht brennbares Zielgas ($CH_4$) im Inneren zu oxidieren vermag und / oder
   - die Gasmessvorrichtung (100) so ausgestaltet ist, dass aus einer Umgebung der Gasmessvorrichtung (100) eine geringere Menge pro Zeiteinheit eines brennbaren Zielgases ($CH_4$) zu dem Kompensator (11) als zu dem Detektor (10) gelangt,

   wobei die Sensor-Anordnung (12.1, 12.2, 13.1, 13.2, 40, 41) dazu ausgestaltet ist, eine erste Detektionsgröße (U10, $\Delta U\_B$) und eine zweite Detektionsgröße (U11) zu messen,
   wobei

   - in einer ersten Alternative die erste Detektionsgröße (U10, $\Delta U\_B$) von der Detektor-Temperatur, das ist die Temperatur des heizenden Detektor-Segments (20), und die zweite Detektionsgröße (U11) von der Kompensator-Temperatur, das ist die Temperatur des heizenden Kompensator-Segments (30), abhängt und
- in einer zweiten Alternative die erste Detektionsgröße (U10, ΔU_B) sowohl von der Detektor-Temperatur als auch von der Kompensator-Temperatur und die zweite Detektionsgröße (U11) entweder von der Detektor-Temperatur, aber nicht von der Kompensator-Temperatur oder von der Kompensator-Temperatur, aber nicht von der Detektor-Temperatur abhängt,

wobei der Temperatur-Sensor (14) dazu ausgestaltet ist, ein Maß für die Umgebungstemperatur in der Umgebung der Gasmessvorrichtung (100) zu messen,
wobei im Datenspeicher (7) ein rechnerauswertbares Modell (Mod) abgespeichert ist,
wobei das abgespeicherte Modell (Mod) einen ersten und einen zweiten funktionalen Zusammenhang umfasst,
wobei der erste funktionale Zusammenhang eine Abhängigkeit zwischen der Zielgas-Konzentration einerseits und der ersten Detektionsgröße (U10, ΔU_B) sowie optional der Umgebungstemperatur und / oder der zweiten Detektionsgröße (U11) andererseits beschreibt,
wobei der zweite funktionale Zusammenhang eine Abhängigkeit zwischen einer ersten weiteren Umgebungsbedingung einerseits und der Umgebungstemperatur sowie der ersten Detektionsgröße (U10, ΔU_B) und / oder der zweiten Detektionsgröße (U11) andererseits beschreibt,
wobei die erste weitere Umgebungsbedingung von der Umgebungstemperatur verschieden ist und bevorzugt die Umgebungsfeuchte ist oder umfasst,
wobei der zweite funktionale Zusammenhang mindestens dann gültig ist, wenn die Konzentration des brennbaren Zielgases ($CH_4$) unterhalb einer vorgegebenen Konzentrations-Schranke liegt,
wobei die Auswerteeinheit (9) dazu ausgestaltet ist, abhängig mindestens von der gemessenen ersten Detektionsgröße (U10, ΔU_B) und optional zusätzlich abhängig von der gemessenen Umgebungstemperatur und / oder der gemessenen zweiten Detektionsgröße (U11) und unter Verwendung des ersten funktionalen Zusammenhangs die Zielgas-Konzentration zu ermitteln,
wobei die Auswerteeinheit (9) weiterhin dazu ausgestaltet ist, automatisch

- zu entscheiden, ob der zweite funktionale Zusammenhang bei der ermittelten Zielgas-Konzentration gültig ist oder nicht, und
- dann, wenn der zweite funktionale Zusammenhang gültig ist,

abhängig mindestens von der gemessenen Umgebungstemperatur sowie abhängig von der ersten Detektionsgröße (U10, ΔU_B) und / oder von der gemessenen zweiten Detektionsgröße (U11) und unter Verwendung des zweiten funktionalen Zusammenhangs die erste weitere Umgebungsbedingung zu ermitteln.

2. Gasmessvorrichtung (100) nach Anspruch 1,
**dadurch gekennzeichnet, dass**

im ersten funktionalen Zusammenhang zusätzlich die erste weitere Umgebungsbedingung auftritt, so dass der erste funktionale Zusammenhang die Abhängigkeit zwischen der Zielgas-Konzentration einerseits und der ersten Detektionsgröße (U10, ΔU_B), der ersten weiteren Umgebungsbedingung sowie optional der Umgebungstemperatur und / oder der zweiten Detektionsgröße (U11) andererseits beschreibt,
wobei die Auswerteeinheit (9) dazu ausgestaltet ist,

- dann, wenn ein von der Auswerteeinheit (9) ermittelter Wert für die erste weitere Umgebungsbedingung vorliegt, diesen Wert für die Ermittlung der Zielgas-Konzentration zu verwenden, und
- ansonsten einen vorgegebenen Standardwert für die erste weitere Umgebungsbedingung.

3. Gasmessvorrichtung (100) nach einem der vorhergehenden Ansprüche,
**dadurch gekennzeichnet, dass**

für die erste weitere Umgebungsbedingung ein Wertebereich vorgegeben ist,
wobei die Gasmessvorrichtung (100) dazu ausgestaltet ist,
dann, wenn die Auswerteeinheit (9) für diese erste weitere Umgebungsbedingung einen Wert ermittelt hat, der außerhalb des vorgegebenen Wertebereichs liegt,

- eine Meldung zu generieren und
- bevorzugt den Schritt auszulösen, dass diese Meldung in einer von einem Menschen wahrnehmbaren Form ausgegeben wird.

4. Gasmessvorrichtung (100) nach einem der vorhergehenden Ansprüche,
**dadurch gekennzeichnet, dass**

die Gasmessvorrichtung (100) dazu ausgestaltet ist, von einem Umgebungsbedingungs-Sensor (16) ein Signal zu empfangen,
wobei der Umgebungsbedingungs-Sensor dazu ausgestaltet ist, ein Maß für die erste weitere Umgebungsbedingung zu messen,
wobei das Signal eine Information über mindestens einen vom Umgebungsbedingungs-Sensor (16) gemessenen Wert der ersten weiteren Umgebungsbedingung umfasst und
wobei die Auswerteeinheit (9) dazu ausgestaltet ist,

- wahlweise einen ersten oder einen zweiten Wert für die erste weitere Umgebungsbedingung zu verwenden und / oder
- den ersten Wert mit dem zweiten Wert zu vergleichen,

wobei der erste Wert von der Auswerteeinheit (9) unter Verwendung des zweiten funktionalen Zusammenhangs ermittelt ist und der zweite Wert von der Auswerteeinheit (9) aus dem empfangenen Signal hergeleitet ist.

5. Anordnung umfassend

- eine Gasmessvorrichtung (100) nach einem der vorhergehenden Ansprüche und
- eine Kalibriervorrichtung (110),
wobei die Kalibriervorrichtung (110) einen Umgebungsbedingungs-Sensor (16) umfasst,
wobei der Umgebungsbedingungs-Sensor (16) dazu ausgestaltet ist, ein Maß für die erste weitere Umgebungsbedingung zu messen,
wobei die Kalibriervorrichtung (110) dazu ausgestaltet ist, eine Stichprobe mit mehreren Stichprobenelementen zu erzeugen,
wobei jedes Stichprobenelement jeweils

- einen von der Sensor-Anordnung gemessenen Wert für die erste Detektionsgröße (U10, $\Delta U\_B$),
- einen von der Sensor-Anordnung gemessenen Wert für die zweite Detektionsgröße (U11),
- einen vom Temperatur-Sensor (14) gemessenen Wert für die Umgebungstemperatur und
- einen vom Umgebungsbedingungs-Sensor (16) gemessenen Wert für die erste weitere Umgebungsbedingung

umfasst,
wobei sich bevorzugt diese vier Werte auf denselben Abtast-Zeitpunkt beziehen und
wobei die Kalibriervorrichtung (110) dazu ausgestaltet ist, automatisch

- unter Verwendung der Stichprobe die beiden funktionalen Zusammenhänge (Mod) zu erzeugen und
- zu bewirken, dass die beiden erzeugten funktionale Zusammenhänge (Mod) im Datenspeicher (7) abgespeichert werden.

6. Gasmessverfahren zum Messen der Konzentration eines brennbaren Zielgases ($CH_4$)

unter Verwendung einer Gasmessvorrichtung (100), die

- einen Detektor (10) mit einem heizenden Detektor-Segment (20),
- einen Kompensator (11) mit einem heizenden Kompensator-Segment (30),
- eine Sensor-Anordnung (12.1, 12.2, 13.1, 13.2, 40, 41) und
- einen Temperatur-Sensor (14)

umfasst,
wobei das Gasmessverfahren die automatisch durchgeführten Schritte umfasst, dass

- der Temperatur-Sensor (14) mindestens einmal ein Maß für die Umgebungstemperatur in der Umgebung der Gasmessvorrichtung (100) misst,
- an den Detektor (10) eine elektrische Spannung (U10) angelegt wird, sodass ein elektrischer Strom (I3, I1) durch das heizende Detektor-Segment (20) fließt und das heizende Detektor-Segment (20) erhitzt wird,

- durch eine Erhitzung des heizenden Detektor-Segments (20) ein im Inneren (5, 8) der Gasmessvorrichtung (100) befindliches brennbares Zielgas (CH$_4$) oxidiert wird,

- an den Kompensator (11) eine elektrische Spannung (U11) angelegt wird, sodass ein elektrischer Strom (I3, I2) durch das heizende Kompensator-Segment (30) fließt und das heizende Kompensator-Segment (30) erhitzt wird,

- der Kompensator (11) in geringerem Umfang als der Detektor (10) oder sogar überhaupt nicht ein brennbares Zielgas (CH$_4$) oxidiert und / oder aus einer Umgebung der Gasmessvorrichtung (100) eine geringere Menge pro Zeiteinheit eines brennbaren Zielgases (CH$_4$) zu dem Kompensator (11) als zu dem Detektor (10) gelangt,

- eine erste Detektionsgröße (U10, ΔU_B) und eine zweite Detektionsgröße (U11) gemessen werden,

wobei in einer ersten Alternative die erste Detektionsgröße (U10, ΔU_B) von der Detektor-Temperatur, das ist die Temperatur des heizenden Detektor-Segments (20), und die zweite Detektionsgröße (U11) von der Kompensator-Temperatur, das ist die Temperatur des heizenden Kompensator-Segments (30), abhängt und

wobei in einer zweiten Alternative die erste Detektionsgröße (U10, ΔU_B) sowohl von der Detektor-Temperatur als auch von der Kompensator-Temperatur und die zweite Detektionsgröße (U11) entweder von der Detektor-Temperatur, aber nicht von der Kompensator-Temperatur oder von der Kompensator-Temperatur, aber nicht von der Detektor-Temperatur abhängt,

wobei ein rechnerauswertbares Modell (Mod) vorgegeben wird,

wobei das abgespeicherte Modell (Mod) einen ersten und einen zweiten funktionalen Zusammenhang umfasst,

wobei der erste funktionale Zusammenhang eine Abhängigkeit zwischen der Zielgas-Konzentration einerseits und der ersten Detektionsgröße (U10, ΔU_B) sowie optional der Umgebungstemperatur und / oder der zweiten Detektionsgröße (U11) andererseits beschreibt,

wobei der zweite funktionale Zusammenhang eine Abhängigkeit zwischen einer ersten weiteren Umgebungsbedingung einerseits und der Umgebungstemperatur sowie der ersten Detektionsgröße (U10, ΔU_B) und / oder der zweiten Detektionsgröße (U11) andererseits beschreibt,

wobei die erste weitere Umgebungsbedingung von der Umgebungstemperatur verschieden ist und bevorzugt die Umgebungsfeuchte ist oder umfasst,

wobei der zweite funktionale Zusammenhang mindestens dann gültig ist, wenn die Konzentration des brenn-baren Zielgases (CH$_4$) unterhalb einer vorgegebenen Konzentrations-Schranke liegt, und

wobei das Gasmessverfahren die weiteren automatisch durchgeführten Schritte umfasst, dass

- abhängig mindestens von der gemessenen ersten Detektionsgröße (U10, ΔU_B) und optional zusätzlich abhängig von der gemessenen Umgebungstemperatur und / oder der gemessenen zweiten Detektionsgröße (U11) und unter Verwendung des ersten funktionalen Zusammenhangs die Zielgas-Konzentration ermittelt wird,

- entschieden wird, ob der zweite funktionale Zusammenhang bei der ermittelten Zielgas-Konzentration gültig ist oder nicht, und

- dann, wenn der zweite funktionale Zusammenhang gültig ist,

abhängig mindestens von der gemessenen Umgebungstemperatur sowie abhängig von der ersten Detektionsgröße (U10, ΔU_B) und / oder von der gemessenen zweiten Detektionsgröße (U11) und unter Verwendung des zweiten funktionalen Zusammenhangs die erste weitere Umgebungsbedingung ermittelt wird.

**Claims**

1. Gas measuring device (100) for measuring the concentration of a combustible target gas (CH$_4$),

wherein the gas measuring device (100) comprises

- a detector (10) having a heating detector segment (20),
- a compensator (11) having a heating compensator segment (30),
- a sensor arrangement (12.1, 12.2, 13.1, 13.2, 40, 41),
- a temperature sensor (14),
- a signal processing evaluation unit (9) and

- a data memory (7) to which the evaluation unit (9) has at least temporary read access,

wherein the gas measuring device (100) is designed to

- apply an electrical voltage (U10) to the detector (10) so that an electrical current (13, I1) flows through the heating detector segment (20) and the heating detector segment (20) is heated, and
- apply an electrical voltage (U11) to the compensator (11) so that an electrical current (13, I2) flows through the heating compensator segment (30) and the heating compensator segment (30) is heated,

wherein the detector (10) is designed to oxidize combustible target gas ($CH_4$) located in the interior of the gas measuring device (100) by heating the heating detector segment (20),
wherein

- the compensator (11) is capable of oxidizing combustible target gas ($CH_4$) in the interior to a lesser extent than the detector (10) or even not at all, and/or
- the gas measuring device (100) is designed such that a smaller quantity per unit of time of a combustible target gas ($CH_4$) reaches the compensator (11) than the detector (10) from the surroundings of the gas measuring device (100),

wherein the sensor arrangement (12.1, 12.2, 13.1, 13.2, 40, 41) is designed to measure a first detection variable (U10, $\Delta U\_B$) and a second detection variable (U11),
wherein

- in a first alternative, the first detection variable (U10, $\Delta U\_B$) depends on the detector temperature, that is the temperature of the heating detector segment (20), and the second detection variable (U11) depends on the compensator temperature, that is the temperature of the heating compensator segment (30), and
- in a second alternative, the first detection variable (U10, $\Delta U\_B$) depends on both the detector temperature and the compensator temperature and the second detection variable (U11) depends either on the detector temperature but not on the compensator temperature or on the compensator temperature but not on the detector temperature,

wherein the temperature sensor (14) is designed to measure a measurement of the surrounding temperature in the surroundings of the gas measuring device (100),
wherein a computer-evaluable model (Mod) is stored in the data memory (7),
wherein the stored model (Mod) comprises a first and a second functional relationship,
wherein the first functional relationship describes a dependency between the target gas concentration on the one hand and the first detection variable (U10, $\Delta U\_B$) and optionally the surrounding temperature and/or the second detection variable (U11) on the other hand,
wherein the second functional relationship describes a dependency between a first further surrounding condition on the one hand and the surrounding temperature and the first detection variable (U10, $\Delta U\_B$) and/or the second detection variable (U11) on the other hand,
wherein the first further surrounding condition is different from the surrounding temperature and preferably is or includes the surrounding humidity,
wherein the second functional relationship is valid at least if the concentration of the combustible target gas ($CH_4$) is below a predetermined concentration threshold,
wherein the evaluation unit (9) is designed to determine the target gas concentration depending at least on the measured first detection variable (U10, $\Delta U\_B$) and optionally additionally depending on the measured surrounding temperature and/or the measured second detection variable (U11) and using the first functional relationship,
wherein the evaluation unit (9) is furthermore designed to automatically

- decide whether or not the second functional relationship is valid at the determined target gas concentration, and
- if the second functional relationship is valid,

determine the first further surrounding condition depending at least on the measured surrounding temperature and depending on the first detection variable (U10, $\Delta U\_B$) and/or on the measured second detection variable (U11) and using the second functional relationship.

**2.** Gas measuring device (100) according to claim 1,
**characterized in that**

the first further surrounding condition also occurs in the first functional relationship, such that the first functional relationship describes the dependency between the target gas concentration on the one hand and the first detection variable (U10, ΔU_B), the first further surrounding condition and optionally the surrounding temperature and/or the second detection variable (U11) on the other hand,
wherein the evaluation unit (9) is designed to

- if a value determined by the evaluation unit (9) for the first further surrounding condition is available, use this value to determine the target gas concentration, and
- otherwise use a predetermined default value for the first further surrounding condition.

**3.** Gas measuring device (100) according to any of the preceding claims,
**characterized in that**

a value range is predetermined for the first further surrounding condition,
wherein the gas measuring device (100) is designed to, if the evaluation unit (9) has determined a value for this first further surrounding condition that lies outside the predetermined value range,

- generate a message and
- preferably trigger the step of outputting this message in a form that can be perceived by a human.

**4.** Gas measuring device (100) according to any of the preceding claims,
**characterized in that**

the gas measuring device (100) is designed to receive a signal from a surrounding condition sensor (16),
wherein the surrounding condition sensor is designed to measure a measurement of the first further surrounding condition,
wherein the signal comprises information about at least one value of the first further surrounding condition measured by the surrounding condition sensor (16) and
wherein the evaluation unit (9) is designed to

- optionally use a first or a second value for the first further surrounding condition and/or
- compare the first value with the second value,

wherein the first value is determined by the evaluation unit (9) using the second functional relationship and the second value is derived by the evaluation unit (9) from the received signal.

**5.** Arrangement comprising

- a gas measuring device (100) according to any of the preceding claims, and
- a calibration device (110),

wherein the calibration device (110) comprises a surrounding condition sensor (16),
wherein the surrounding condition sensor (16) is designed to measure a measurement of the first further surrounding condition,
wherein the calibration device (110) is designed to generate a sample comprising a plurality of sample elements,
wherein each sample element comprises

- a value measured by the sensor arrangement for the first detection variable (U10, ΔU_B),
- a value measured by the sensor arrangement for the second detection variable (U11),
- a value measured by the temperature sensor (14) for the surrounding temperature and
- a value measured by the surrounding condition sensor (16) for the first further surrounding condition,

wherein these four values preferably refer to the same sampling time and
wherein the calibration device (110) is designed to automatically

- generate the two functional relationships (Mod) using the sample and
- ensure that the two generated functional relationships (Mod) are stored in the data memory (7).

**6.** Gas measurement method for measuring the concentration of a combustible target gas ($CH_4$) using a gas measuring device (100) comprising

- a detector (10) having a heating detector segment (20),
- a compensator (11) having a heating compensator segment (30),
- a sensor arrangement (12.1, 12.2, 13.1, 13.2, 40, 41) and
- a temperature sensor (14),

wherein the gas measurement method comprises the automatically performed steps of

- the temperature sensor (14) measuring at least once a measurement of the surrounding temperature in the surroundings of the gas measuring device (100),
- an electrical voltage (U10) being applied to the detector (10) so that an electrical current (13, 11) flows through the heating detector segment (20) and the heating detector segment (20) is heated,
- a combustible target gas ($CH_4$) located in the interior (5, 8) of the gas measuring device (100) being oxidized by heating the heating detector segment (20),
- an electrical voltage (U11) being applied to the compensator (11) so that an electrical current (13, 12) flows through the heating compensator segment (30) and the heating compensator segment (30) is heated,
- the compensator (11) oxidizing a combustible target gas ($CH_4$) to a lesser extent than the detector (10) or even not at all and/or a smaller quantity per unit of time of a combustible target gas ($CH_4$) reaching the compensator (11) than the detector (10) from the surroundings of the gas measuring device (100),
- a first detection variable (U10, $\Delta U\_B$) and a second detection variable (U11) being measured,

wherein, in a first alternative, the first detection variable (U10, $\Delta U\_B$) depends on the detector temperature, that is the temperature of the heating detector segment (20), and the second detection variable (U11) depends on the compensator temperature, that is the temperature of the heating compensator segment (30), and

wherein, in a second alternative, the first detection variable (U10, $\Delta U\_B$) depends on both the detector temperature and the compensator temperature and the second detection variable (U11) depends either on the detector temperature but not on the compensator temperature or on the compensator temperature but not on the detector temperature,

wherein a computer-evaluable model (Mod) is predetermined,

wherein the stored model (Mod) comprises a first and a second functional relationship,

wherein the first functional relationship describes a dependency between the target gas concentration on the one hand and the first detection variable (U10, $\Delta U\_B$) and optionally the surrounding temperature and/or the second detection variable (U11) on the other hand,

wherein the second functional relationship describes a dependency between a first further surrounding condition on the one hand and the surrounding temperature and the first detection variable (U10, $\Delta U\_B$) and/or the second detection variable (U11) on the other hand,

wherein the first further surrounding condition is different from the surrounding temperature and preferably is or includes the surrounding humidity,

wherein the second functional relationship is valid at least if the concentration of the combustible target gas ($CH_4$) is below a predetermined concentration threshold, and

wherein the gas measurement method comprises the further automatically performed steps of

- the target gas concentration being determined depending at least on the measured first detection variable (U10, $\Delta U\_B$) and optionally additionally depending on the measured surrounding temperature and/or the measured second detection variable (U11) and using the first functional relationship,
- it being decided whether or not the second functional relationship is valid at the determined target gas concentration, and
- if the second functional relationship is valid,

the first further surrounding condition being determined depending at least on the measured surrounding temperature and depending on the first detection variable (U10, $\Delta U\_B$) and/or on the measured second detection variable (U11) and using the second functional relationship.

**Revendications**

1. Dispositif de mesure de gaz (100) permettant de mesurer la concentration d'un gaz cible combustible ($CH_4$),

   dans lequel le dispositif de mesure de gaz (100) comprend

   - un détecteur (10) comportant un segment de détecteur chauffant (20),
   - un compensateur (11) comportant un segment de compensateur chauffant (30),
   - un système de capteurs (12.1, 12.2, 13.1, 13.2, 40, 41),
   - un capteur de température (14),
   - une unité d'évaluation (9) traitant des signaux, et
   - une mémoire de données (7) à laquelle l'unité d'évaluation (9) a accès en lecture au moins temporairement,

   dans lequel le dispositif de mesure de gaz (100) est configuré pour

   - appliquer une tension électrique (U10) au détecteur (10) de sorte qu'un courant électrique (13, 11) circule à travers le segment de détecteur chauffant (20) et que le segment de détecteur chauffant (20) est chauffé, et
   - appliquer une tension électrique (U11) au compensateur (11) de sorte qu'un courant électrique (13, 12) circule à travers le segment de compensateur chauffant (30) et que le segment de compensateur chauffant (30) est chauffé,

   dans lequel le détecteur (10) est configuré pour oxyder le gaz cible combustible ($CH_4$) se trouvant à l'intérieur du dispositif de mesure de gaz (100) par un chauffage du segment de détecteur chauffant (20),
   dans lequel

   - le compensateur (11) est capable dans une moindre mesure que le détecteur (10), voire n'est pas du tout capable, d'oxyder le gaz cible combustible ($CH_4$) à l'intérieur, et/ou
   - le dispositif de mesure de gaz (100) est configuré de sorte que, en provenance d'un environnement du dispositif de mesure de gaz (100), par unité de temps, une quantité d'un gaz cible combustible ($CH_4$) atteignant le compensateur (11) est plus faible que celle atteignant le détecteur (10),

   dans lequel le système de capteurs (12.1, 12.2, 13.1, 13.2, 40, 41) est configuré pour mesurer une première grandeur de détection (U10, ΔU_B) et une seconde grandeur de détection (U11),
   dans lequel

   - dans une première alternative, la première grandeur de détection (U10, ΔU_B) dépend de la température de détecteur qui est la température du segment de détecteur chauffant (20), et la seconde grandeur de détection (U11) dépend de la température de compensateur qui est la température du segment de compensateur chauffant (30), et
   - dans une seconde alternative, la première grandeur de détection (U10, ΔU_B) dépend à la fois de la température de détecteur et de la température de compensateur, et la seconde grandeur de détection (U11) dépend soit de la température de détecteur, mais pas de la température de compensateur, soit de la température de compensateur, mais pas de la température de détecteur,

   dans lequel le capteur de température (14) est configuré pour mesurer une mesure de la température d'environnement dans l'environnement du dispositif de mesure de gaz (100),
   dans lequel un modèle (Mod) exploitable par ordinateur est mémorisé dans la mémoire de données (7),
   dans lequel le modèle (Mod) mémorisé comprend une première et une seconde relation fonctionnelle,
   dans lequel la première relation fonctionnelle décrit une dépendance entre la concentration de gaz cible d'une part et la première grandeur de détection (U10, ΔU_B) ainsi qu'éventuellement la température d'environnement et/ou la seconde grandeur de détection (U11) d'autre part,
   dans lequel la seconde relation fonctionnelle décrit une dépendance entre une première autre condition d'environnement d'une part et la température d'environnement ainsi que la première grandeur de détection (U10, ΔU_B) et/ou la seconde grandeur de détection (U11) d'autre part,
   dans lequel la première autre condition d'environnement est différente de la température d'environnement et est ou comprend de préférence l'humidité d'environnement,

dans lequel la seconde relation fonctionnelle est valable au moins lorsque la concentration du gaz cible combustible ($CH_4$) est inférieure à une limite de concentration prédéfinie,

dans lequel l'unité d'évaluation (9) est configurée pour déterminer la concentration de gaz cible en fonction d'au moins la première grandeur de détection (U10, $\Delta U\_B$) mesurée et, éventuellement, également en fonction de la température d'environnement mesurée et/ou de la seconde grandeur de détection (U11) mesurée et en utilisant la première relation fonctionnelle,

dans lequel l'unité d'évaluation (9) est en outre configurée pour

- décider automatiquement si la seconde relation fonctionnelle est valable ou non pour la concentration de gaz cible déterminée, et
- si la seconde relation fonctionnelle est valable,

déterminer automatiquement la première autre condition d'environnement en fonction d'au moins la température d'environnement mesurée ainsi qu'en fonction de la première grandeur de détection (U10, $\Delta U\_B$) et/ou de la seconde grandeur de détection (U11) mesurée et en utilisant la seconde relation fonctionnelle.

2. Dispositif de mesure de gaz (100) selon la revendication 1,
**caractérisé en ce que**

dans la première relation fonctionnelle, la première autre condition d'environnement apparaît en outre, de sorte que la première relation fonctionnelle décrit la dépendance entre la concentration de gaz cible d'une part et la première grandeur de détection (U10, $\Delta U\_B$), la première autre condition d'environnement ainsi que, éventuellement, la température d'environnement et/ou la seconde grandeur de détection (U11) d'autre part,

dans lequel l'unité d'évaluation (9) est configurée pour

- lorsqu'une valeur déterminée par l'unité d'évaluation (9) pour la première autre condition d'environnement existe, utiliser ladite valeur pour la détermination de la concentration de gaz cible, et
- sinon, une valeur standard prédéfinie pour la première autre condition d'environnement.

3. Dispositif de mesure de gaz (100) selon l'une des revendications précédentes,
**caractérisé en ce que**

une plage de valeurs est prédéfinie pour la première autre condition d'environnement,

dans lequel le dispositif de mesure de gaz (100) est configuré pour, lorsque l'unité d'évaluation (9) a déterminé pour ladite première autre condition d'environnement une valeur qui se situe en dehors de la plage de valeurs prédéfinie,

- générer un message, et
- de préférence, déclencher l'étape selon laquelle ledit message est émis sous une forme perceptible par un être humain.

4. Dispositif de mesure de gaz (100) selon l'une des revendications précédentes,
**caractérisé en ce que**

le dispositif de mesure de gaz (100) est configuré pour recevoir un signal en provenance d'un capteur de condition d'environnement (16),

dans lequel le capteur de condition d'environnement est configuré pour mesurer une mesure de la première autre condition d'environnement,

dans lequel le signal comprend des informations concernant au moins une valeur de la première autre condition d'environnement, laquelle valeur est mesurée par le capteur de condition d'environnement (16), et

dans lequel l'unité d'évaluation (9) est configurée pour

- utiliser au choix une première ou une seconde valeur pour la première autre condition d'environnement, et/ou
- comparer la première valeur avec la seconde valeur,

dans lequel la première valeur est déterminée par l'unité d'évaluation (9) en utilisant la seconde relation fonctionnelle et la seconde valeur est déduite par l'unité d'évaluation (9) à partir du signal reçu.

5. Système comprenant

- un dispositif de mesure de gaz (100) selon l'une des revendications précédentes, et
- un dispositif de calibrage (110),

dans lequel le dispositif de calibrage (110) comprend un capteur de condition d'environnement (16),
dans lequel le capteur de condition d'environnement (16) est configuré pour mesurer une mesure de la première autre condition d'environnement,
dans lequel le dispositif de calibrage (110) est configuré pour créer un échantillon comportant plusieurs éléments d'échantillon,
dans lequel chaque élément d'échantillon comprend respectivement

- une valeur mesurée par le système de capteurs pour la première grandeur de détection (U10, ∆U_B),
- une valeur mesurée par le système de capteurs pour la seconde grandeur de détection (U11),
- une valeur pour la température d'environnement, laquelle valeur est mesurée par le capteur de température (14), et
- une valeur pour la première autre condition d'environnement, laquelle valeur est mesurée par le capteur de condition d'environnement (16),

dans lequel, de préférence, lesdites quatre valeurs se rapportent au même instant de balayage, et
dans lequel le dispositif de calibrage (110) est configuré pour

- créer automatiquement les deux relations fonctionnelles (Mod) en utilisant l'échantillon, et
- faire automatiquement en sorte que les deux relations fonctionnelles (Mod) créées soient mémorisées dans la mémoire de données (7).

6. Procédé de mesure de gaz permettant de mesurer la concentration d'un gaz cible combustible (CH$_4$) en utilisant un dispositif de mesure de gaz (100) qui comprend

- un détecteur (10) comportant un segment de détecteur chauffant (20),
- un compensateur (11) comportant un segment de compensateur chauffant (30),
- un système de capteurs (12.1, 12.2, 13.1, 13.2, 40, 41), et
- un capteur de température (14),

dans lequel le procédé de mesure de gaz comprend les étapes exécutées automatiquement, selon lesquelles

- le capteur de température (14) mesure au moins une fois une mesure de la température d'environnement dans l'environnement du dispositif de mesure de gaz (100),
- une tension électrique (U10) est appliquée au détecteur (10) de sorte qu'un courant électrique (13, 11) circule à travers le segment de détecteur chauffant (20) et que le segment de détecteur chauffant (20) est chauffé,
- un gaz cible combustible (CH$_4$) se trouvant à l'intérieur (5, 8) du dispositif de mesure de gaz (100) est oxydé par un chauffage du segment de détecteur chauffant (20),
- une tension électrique (U11) est appliquée au compensateur (11) de sorte qu'un courant électrique (13, 12) circule à travers le segment de compensateur chauffant (30) et que le segment de compensateur chauffant (30) est chauffé,
- le compensateur (11) oxyde un gaz cible combustible (CH$_4$) dans une moindre mesure que le détecteur (10), voire pas du tout, et/ou, en provenance d'un environnement du dispositif de mesure de gaz (100), par unité de temps, une quantité d'un gaz cible combustible (CH$_4$) atteignant le compensateur (11) est plus faible que celle atteignant le détecteur (10),
- une première grandeur de détection (U10, ∆U_B) et une seconde grandeur de détection (U11) sont mesurées,

dans lequel, dans une première alternative, la première grandeur de détection (U10, ∆U_B) dépend de la température de détecteur qui est la température du segment de détecteur chauffant (20), et la seconde grandeur de détection (U11) dépend de la température de compensateur qui est la température du segment de compensateur chauffant (30), et

dans lequel, dans une seconde alternative, la première grandeur de détection (U10, ΔU_B) dépend à la fois de la température de détecteur et de la température de compensateur, et la seconde grandeur de détection (U11) dépend soit de la température de détecteur, mais pas de la température de compensateur, soit de la température de compensateur, mais pas de la température de détecteur,

dans lequel un modèle (Mod) exploitable par ordinateur est prédéfini,

dans lequel le modèle (Mod) mémorisé comprend une première et une seconde relation fonctionnelle,

dans lequel la première relation fonctionnelle décrit une dépendance entre la concentration de gaz cible d'une part et la première grandeur de détection (U10, ΔU_B) ainsi qu'éventuellement la température d'environnement et/ou la seconde grandeur de détection (U11) d'autre part,

dans lequel la seconde relation fonctionnelle décrit une dépendance entre une première autre condition d'environnement d'une part et la température d'environnement ainsi que la première grandeur de détection (U10, ΔU_B) et/ou la seconde grandeur de détection (U11) d'autre part,

dans lequel la première autre condition d'environnement est différente de la température d'environnement et est ou comprend de préférence l'humidité d'environnement,

dans lequel la seconde relation fonctionnelle est valable au moins lorsque la concentration du gaz cible combustible ($CH_4$) est inférieure à une limite de concentration prédéfinie, et

dans lequel le procédé de mesure de gaz comprend les autres étapes exécutées automatiquement, selon lesquelles

- en fonction d'au moins la première grandeur de détection (U10, ΔU_B) mesurée et, éventuellement, également en fonction de la température d'environnement mesurée et/ou de la seconde grandeur de détection (U11) mesurée et en utilisant la première relation fonctionnelle, la concentration de gaz cible est déterminée,
- on décide si la seconde relation fonctionnelle est valable ou non pour la concentration de gaz cible déterminée, et
- si la seconde relation fonctionnelle est valable,

la première autre condition d'environnement est déterminée en fonction d'au moins la température d'environnement mesurée ainsi qu'en fonction de la première grandeur de détection (U10, ΔU_B) et/ou de la seconde grandeur de détection (U11) mesurée et en utilisant la seconde relation fonctionnelle.

FIG. 1

EP 4 321 859 B1

FIG. 2

FIG. 3

EP 4 321 859 B1

FIG. 4

EP 4 321 859 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3808305 C2
- DE 4130099 A1
- DE 102007021957 A1
- US 2008282771 A1